(19) Europäisches Patentamt
European Patent Office
Office européen des brevets

(11) EP 3 090 051 B1

# (12) FASCICULE DE BREVET EUROPEEN

(45) Date de publication et mention de la délivrance du brevet:
**03.04.2019 Bulletin 2019/14**

(21) Numéro de dépôt: **14824856.0**

(22) Date de dépôt: **30.12.2014**

(51) Int Cl.:
***C12N 15/115*** *(2010.01)*

(86) Numéro de dépôt international:
**PCT/EP2014/079471**

(87) Numéro de publication internationale:
**WO 2015/101637 (09.07.2015 Gazette 2015/27)**

(54) **APTAMERES INHIBITEURS DE L'ACTIVITE ENZYMATIQUE DE LA PROTEINE MMP-9**
APTAMERE ZUR HEMMUNG DER ENZYMATISCHEN AKTIVITÄT DES MMP-9-PROTEINS
APTAMERS INHIBITING THE ENZYMATIC ACTIVITY OF THE MMP-9 PROTEIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.12.2013 FR 1363696**

(43) Date de publication de la demande:
**09.11.2016 Bulletin 2016/45**

(73) Titulaires:
- **L V M H RECHERCHE**
  **45800 St. Jean de Braye (FR)**
- **INSTITUT NATIONAL DE LA SANTE ET DE LA RECHERCHE MEDICALE (INSERM)**
  **75013 Paris (FR)**

(72) Inventeurs:
- **DAUSSE, Eric**
  **F-33000 Bordeaux (FR)**
- **TOULME, Jean-Jacques**
  **F-33310 Lormont (FR)**
- **CAUCHARD, Jean Hubert**
  **F-45000 Orleans (FR)**
- **KURFURST, Robin**
  **F-45800 Saint Jean de Braye (FR)**
- **SCHNEBERT, Sylvianne**
  **F-45160 Olivet (FR)**

(74) Mandataire: **Regimbeau**
**20, rue de Chazelles**
**75847 Paris Cedex 17 (FR)**

(56) Documents cités:
**WO-A1-2013/153138 WO-A2-2010/110914**

- **Z. GU: "A Highly Specific Inhibitor of Matrix Metalloproteinase-9 Rescues Laminin from Proteolysis and Neurons from Apoptosis in Transient Focal Cerebral Ischemia", JOURNAL OF NEUROSCIENCE, vol. 25, no. 27, 6 juillet 2005 (2005-07-06), pages 6401-6408, XP055130459, ISSN: 0270-6474, DOI: 10.1523/JNEUROSCI.1563-05.2005**
- **GATTO B ET AL: "Nucleic Acid Aptamers Based on the G-Quadruplex Structure: Therapeutic and Diagnostic Potential", CURRENT MEDICINAL CHEMISTRY, BENTHAM SCIENCE PUBLISHERS, NL, vol. 16, no. 10, 1 avril 2009 (2009-04-01) , pages 1248-1265, XP002634830, ISSN: 0929-8673, DOI: 10.2174/092986709787846640**
- **W. O. TUCKER ET AL: "G-quadruplex DNA Aptamers and their Ligands: Structure, Function and Application", CURRENT PHARMACEUTICAL DESIGN, vol. 18, no. 14, 1 mars 2012 (2012-03-01), pages 2014-2026, XP055130400, ISSN: 1381-6128, DOI: 10.2174/138161212799958477**
- **SONIA DA ROCHA GOMES ET AL: "99m Tc-MAG3-Aptamer for Imaging Human Tumors Associated with High Level of Matrix Metalloprotease-9", BIOCONJUGATE CHEMISTRY, vol. 23, no. 11, 21 novembre 2012 (2012-11-21), pages 2192-2200, XP055130401, ISSN: 1043-1802, DOI: 10.1021/bc300146c**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> L V M H RECHERCHE INSTITUT NATIONAL DE LA SANTE ET DE LA RECHERCHE MEDICALE (INSERM)

<120> APTAMERES INHIBITEURS DE l’ACTIVITE ENZYMATIQUE DE LA PROTEINE MMP-9

<130> 366583 D29494

<150> FR1363696
<151> 2013-12-30

<160> 61

<170> PatentIn version 3.5

<210> 1
<211> 27
<212> DNA
<213> artificial

<220>
<223> séquence consensus aptamère anti-MMP9 Groupe I

<220>

<221> misc_feature
<222> (1)..(4)
<223> n représente 0 ou 1 nucléotide G

<220>
<221> misc_feature
<222> (26).. (27)
<223> n représente 0 ou 1 nucléotide G

<400> 1
nnnntttggt gggtytgggg twgyknn 27

<210> 2
<211> 23
<212> DNA
<213> artificial

<220>
<223> séquence consensus aptamère anti-MMP9 Groupe II

<400> 2
tggcrcgggg ttggtgtygg gtt 23

<210> 3
<211> 24
<212> DNA
<213> artificial

<220>
<223> séquence consensus aptamère anti-MMP9 Groupe III

<220>
<221> misc_feature
<222> (12)..(12)
<223> n représente 0 ou 1 nucléotide A

<400> 3
gggwttggct tncggygcct ggcg 24

<210> 4
<211> 21
<212> DNA
<213> artificial

<220>
<223> séquence consensus aptamère anti-MMP9 Groupe IV

<220>
<221> misc_feature
<222> (9)..(9)
<223> n représente 0 ou 1 nucléotide T

<400> 4
gtggttggng skrtrgwkgk t 21

<210> 5
<211> 14
<212> DNA

<213> artificial

<220>
<223> séquence consensus aptamère anti-MMP9 Groupe V

<400> 5
gggtgggggg gtgg 14

<210> 6
<211> 30
<212> DNA
<213> artificial

<220>
<223> séquence consensus aptamère anti-MMP9 Groupe VI

<400> 6
ttggtgggat gggggggggg ttgttcggct 30

<210> 7
<211> 30
<212> DNA
<213> artificial

<220>
<223> séquence consensus aptamère anti-MMP9 Groupe VII

<400> 7
ctgggggtgt gtygcgattg tgtgggtggg 30

<210> 8
<211> 30
<212> DNA
<213> artificial

<220>
<223> séquence consensus aptamère anti-MMP9 Groupe VIII

<400> 8
scscggtgga ytggttgggt ttggatcccc 30

<210> 9
<211> 30
<212> DNA
<213> artificial

<220>
<223> séquence consensus aptamère anti-MMP9 Groupe IX

<400> 9
tgaggggggt ggatgggagg gttccgcacg 30

<210> 10
<211> 30
<212> DNA
<213> artificial

<220>

<223> séquence consensus aptamère anti-MMP9 Groupe X

<400> 10
tggacggtgg gttggggcgg ggggtgtcca 30

<210> 11
<211> 21
<212> DNA
<213> artificial

<220>
<223> aptamère 11F46min

<400> 11
tttggtgggt ctggggttgc t 21

<210> 12
<211> 30
<212> DNA
<213> artificial

<220>
<223> aptamère 11F46R

<400> 12
tggggtttgg tgggtctggg gttgctggcc 30

<210> 13
<211> 30
<212> DNA
<213> artificial

<220>
<223> aptamère 11F76R

<400> 13
tggggtttgg tgggtttggg gttgctggcc 30

<210> 14
<211> 30
<212> DNA
<213> artificial

<220>
<223> aptamère 11F89R

<400> 14
tagggtttgg tgggtctggg gttgctggcc 30

<210> 15
<211> 30
<212> DNA
<213> artificial

<220>
<223> aptamère 11F57R

<400> 15

ccggggtttg gtgggtctgg ggtagttggc 30

<210> 16
<211> 30
<212> DNA
<213> artificial

<220>
<223> aptamère 8F68R

<400> 16
ttggggtttg gtgggtctgg ggttgcgggt 30

<210> 17
<211> 30
<212> DNA
<213> artificial

<220>
<223> aptamère 8F44R

<400> 17
gcggggtttg gtgggtctgg ggttgttggt 30

<210> 18
<211> 25
<212> DNA
<213> artificial

<220>
<223> aptamère 8F14min

<400> 18
tatggcacgg ggttggtgtt gggtt 25

<210> 19
<211> 30
<212> DNA
<213> artificial

<220>
<223> aptamère 8F14R

<400> 19
tcgtatggca cggggttggt gttgggttgg 30

<210> 20
<211> 30
<212> DNA
<213> artificial

<220>
<223> aptamère 8F19R

<400> 20
ctggcgcggg gttggtgtcg ggtttggttt 30

<210> 21

<211> 27
<212> DNA
<213> artificial

<220>
<223> aptamère 8F27min

<400> 21
cgagggtttg gcttacggcg cctggcg 27

<210> 22
<211> 30
<212> DNA
<213> artificial

<220>
<223> aptamère 8F27R

<400> 22
ccgcgagggt ttggcttacg gcgcctggcg 30

<210> 23
<211> 30
<212> DNA
<213> artificial

<220>
<223> aptamère 8F50R

<400> 23
ccgttgggat tggcttcggt gcctggcgtg 30

<210> 24
<211> 29
<212> DNA
<213> artificial

<220>
<223> aptamère 8F11R

<400> 24
ggtggttggt ggggtggagg ttaggtacc 29

<210> 25
<211> 30
<212> DNA
<213> artificial

<220>
<223> aptamère 8F70R

<400> 25
mgtagtggtt gggctgtagt ggttgggacc 30

<210> 26
<211> 27
<212> DNA
<213> artificial

<220>
<223> aptamère 8F77min

<400> 26
gtggttgggg tatggttggt acaggtt 27

<210> 27
<211> 30
<212> DNA
<213> artificial

<220>
<223> aptamère 8F77R

<400> 27
gaagtggttg gggtatggtt ggtacaggtt 30

<210> 28
<211> 30
<212> DNA
<213> artificial

<220>
<223> aptamère 8F21R

<400> 28
tggctggyga ccttgcgggt gggggggtgg 30

<210> 29
<211> 30
<212> DNA
<213> artificial

<220>
<223> aptamère 8F67R

<400> 29
cctgcgccgt gattaggggt gggggggtgg 30

<210> 30
<211> 24
<212> DNA
<213> artificial

<220>
<223> région fixe 5\222

<400> 30
gcctgttgtg agcctcctgt cgaa 24

<210> 31
<211> 23
<212> DNA
<213> artificial

<220>
<223> région fixe 3’

<400> 31
ttgagcgttt attcttgtct ccc 23

<210> 32
<211> 41
<212> DNA
<213> artificial

<220>
<223> aptamère 11F46A

<400> 32
tcgaatgggg tttggtgggt ctggggttgc tggccttgag c 41

<210> 33
<211> 31
<212> DNA
<213> artificial

<220>
<223> aptamère 11F46B

<400> 33
tcgaatgggg tttggtgggt ctggggttgc t 31

<210> 34
<211> 31
<212> DNA
<213> artificial

<220>
<223> aptamère 11F46C

<400> 34
tttggtgggt ctggggttgc tggccttgag c 31

<210> 35
<211> 41
<212> DNA
<213> artificial

<220>
<223> aptamère 8F14A

<400> 35
tcgaatcgta tggcacgggg ttggtgttgg gttggttgag c 41

<210> 36
<211> 33
<212> DNA
<213> artificial

<220>
<223> aptamère 8F14B

<400> 36
tcgaatcgta tggcacgggg ttggtgttgg gtt 33

<210> 37
<211> 33
<212> DNA
<213> artificial

<220>
<223> aptamère 8F14C

<400> 37
tatggcacgg ggttggtgtt gggttggttg agc 33

<210> 38
<211> 41
<212> DNA
<213> artificial

<220>
<223> aptamère 8F27A

<400> 38
tcgaaccgcg agggtttggc ttacggcgcc tggcgttgag c 41

<210> 39
<211> 28
<212> DNA
<213> artificial

<220>
<223> aptamère 8F27B

<400> 39
cgagggtttg gcttacggcg cctggcgt 28

<210> 40
<211> 77
<212> DNA
<213> artificial

<220>
<223> aptamère 11F46

<400> 40

**gcctgttgtg agcctcctgt cgaatggggt ttggtgggtc tggggttgct ggccttgagc 60**

**gtttattctt gtctccc 77**

<210> 41
<211> 77
<212> DNA
<213> artificial

<220>
<223> aptamère 11F76

<400> 41

gcctgttgtg agcctcctgt cgaatggggt ttggtgggtt tggggttgct ggccttgagc 60

gtttattctt gtctccc 77

<210> 42
<211> 77
<212> DNA
<213> artificial

<220>
<223> aptamère 11F89

<400> 42

gcctgttgtg agcctcctgt cgaatagggt ttggtgggtc tggggttgct ggccttgagc 60

gtttattctt gtctccc 77

<210> 43
<211> 77
<212> DNA
<213> artificial

<220>
<223> aptamère 11F57

<400> 43

gcctgttgtg agcctcctgt cgaaccgggg tttggtgggt ctggggtagt tggcttgagc 60

gtttattctt gtctccc 77

<210> 44
<211> 77
<212> DNA
<213> artificial

<220>
<223> aptamère 8F68

<400> 44

gcctgttgtg agcctcctgt cgaattgggg tttggtgggt ctggggttgc gggtttgagc 60

gtttattctt gtctccc 77

<210> 45
<211> 77
<212> DNA
<213> artificial

<220>
<223> aptamère 8F44

<400> 45

gcctgttgtg agcctcctgt cgaagcgggg tttggtgggt ctggggttgt tggtttgagc 60

gtttattctt gtctccc 77

<210> 46
<211> 77
<212> DNA
<213> artificial

<220>
<223> aptamère 8F14

<400> 46

gcctgttgtg agcctcctgt cgaatcgtat ggcacggggt tggtgttggg ttggttgagc 60

gtttattctt gtctccc 77

<210> 47
<211> 77
<212> DNA
<213> artificial

<220>
<223> aptamère 8F19

<400> 47

gcctgttgtg agcctcctgt cgaactggcg cggggttggt gtcgggtttg gtttttgagc 60

gtttattctt gtctccc 77

<210> 48
<211> 77
<212> DNA
<213> artificial

<220>
<223> aptamère 8F27

<400> 48

gcctgttgtg agcctcctgt cgaaccgcga gggtttggct tacggcgcct ggcgttgagc 60

gtttattctt gtctccc 77

<210> 49
<211> 77
<212> DNA
<213> artificial

<220>
<223> aptamère 8F50

<400> 49

gcctgttgtg agcctcctgt cgaaccgttg ggattggctt cggtgcctgg cgtgttgagc 60

gtttattctt gtctccc 77

<210> 50
<211> 77
<212> DNA
<213> artificial

<220>
<223> aptamère 8F11

<400> 50

gcctgttgtg agcctcctgt cgaatggtgg ttggtggggt ggaggttagg taccttgagc 60

gtttattctt gtctccc 77

<210> 51
<211> 77
<212> DNA
<213> artificial

<220>
<223> aptamère 8F70

<400> 51

gcctgttgtg agcctcctgt cgaamgtagt ggttgggctg tagtggttgg gaccttgagc 60

gtttattctt gtctccc 77

<210> 52
<211> 77
<212> DNA
<213> artificial

<220>
<223> aptamère 8F77

<400> 52

gcctgttgtg agcctcctgt cgaagaagtg gttggggtat ggttggtaca ggttttgagc 60

gtttattctt gtctccc 77

<210> 53
<211> 77
<212> DNA
<213> artificial

<220>
<223> aptamère 8F21

<400> 53

```
gcctgttgtg agcctcctgt cgaatggctg gygaccttgc gggtgggggg gtggttgagc    60
gtttattctt gtctccc                                                   77
```

<210> 54
<211> 77
<212> DNA
<213> artificial

<220>
<223> aptamère 8F67

<400> 54

```
gcctgttgtg agcctcctgt cgaacctgcg ccgtgattag gggtgggggg gtggttgagc    60
gtttattctt gtctccc                                                   77
```

<210> 55
<211> 77
<212> DNA
<213> artificial

<220>
<223> aptamère 8F5

<400> 55

```
gcctgttgtg agcctcctgt cgaattggtg ggatgggggg ggggttgttc ggctttgagc    60
gtttattctt gtctccc                                                   77
```

<210> 56
<211> 77
<212> DNA
<213> artificial

<220>
<223> aptamère 8F9

<400> 56

```
gcctgttgtg agcctcctgt cgaactgggg gtgtgtygcg attgtgtggg tgggttgagc    60
gtttattctt gtctccc                                                   77
```

<210> 57
<211> 77
<212> DNA
<213> artificial

<220>
<223> aptamère 8F60

<400> 57

gcctgttgtg agcctcctgt cgaascscgg tggaytggtt gggtttggat ccccttgagc 60

gtttattctt gtctccc 77

<210> 58
<211> 77
<212> DNA
<213> artificial

<220>
<223> aptamère 8F65

<400> 58

gcctgttgtg agcctcctgt cgaatgaggg gggtggatgg gagggttccg cacgttgagc 60

gtttattctt gtctccc 77

<210> 59
<211> 77
<212> DNA
<213> artificial

<220>
<223> aptamère 11F2

<400> 59

gcctgttgtg agcctcctgt cgaatggacg gtgggttggg gcggggggtg tccattgagc 60

gtttattctt gtctccc 77

<210> 60
<211> 23
<212> DNA
<213> artificial

<220>
<223> amorce P3

<400> 60
gggagacaag aataaacgct caa 23

<210> 61
<211> 44
<212> DNA
<213> artificial

<220>
<223> amorce P5

<400> 61
actgactgac tgactgacta cgggagacaa gaataaacgc tcaa 44

## Description

La présente invention concerne un nouvel inhibiteur de métalloprotéase matricielle à haute spécificité, et son utilisation dans des compositions cosmétiques ou pharmaceutiques, notamment en tant qu'agent actif pour lutter contre le vieillissement extrinsèque et/ou intrinsèque de la peau et/ou le développement du tissu adipeux, par l'inhibition de la dégradation des matrices extracellulaires épidermique et/ou dermique et/ou hypodermique.

Les agressions externes telles que les rayons UV, le soleil, les stress oxydatif et hydrique, thermique ou encore les agents xénobiotiques sont des facteurs impliqués dans le remodelage cutané et le processus de vieillissement de la peau, notamment par le déclenchement de réactions inflammatoires via la libération de cytokines induisant la production de métalloprotéases matricielles (MMP).

Les MMPs sont des protéases associées à la dégradation et à la reconstitution des protéines constituant les matrices extracellulaires. Au moins 11 types de MMPs humaines sont connus. On retrouve parmi celles-ci des collagénases (MMP-1, MMP-8 et MMP-13) et des gélatinases (MMP-2 et MMP-9). Les MMPs sont différentes en termes de substrat et de site d'expression.

Les gélatinases MMP-2 et MMP-9 sont connues pour décomposer les constituants de la membrane basale tels que le collagène de type IV, V, la laminine, et l'élastine qui jouent un rôle important dans le maintien de la structure de la peau. La production de ces métalloprotéases, provoquée par des cytokines comme le TGF-$\beta$, entraîne la réduction et la dégénérescence des matrices extracellulaires, phénomènes considérés comme un facteur important de la modification des propriétés physiques de la peau (**Inomata** et al., 2003, 120). L'accumulation de ces dommages est une des principales causes de la formation des rides, de la disparition de la texture de la peau et de la réduction de son élasticité.

Afin de prévenir l'apparition de signes, visibles ou non, du vieillissement cutané, et en atténuer l'évolution, il est donc important de contrôler l'activité de ces métalloprotéases dans la peau.

Les kératinocytes, qui constituent l'épiderme exposé aux UV et autres facteurs extérieurs déclencheurs d'inflammation, expriment la MMP-9. Ainsi, il a été envisagé de développer des inhibiteurs spécifiques de la MMP-9.

On connaît des inhibiteurs de MMPs, qui présentent cependant des inconvénients qui limitent leur intérêt et leur utilisation au niveau cutané.

Certains agents chélatants comme l'EDTA et la o-phénanthroline inhibent le centre actif métallique des MMPs, mais présentent une faible spécificité et sont cytotoxiques. De tels agents ne peuvent donc être appliqués directement sur la peau.

D'autres inhibiteurs de faible poids moléculaire comme des peptides ont été utilisés. Cependant, ils ont montré que leur utilisation dans les produits cosmétiques peut induire des effets secondaires au niveau cutané (**Bertin** et al, 2005).

L'acide rétinoïque a été également utilisé en tant qu'inhibiteur de la MMP-9 (**Lateef** et al., 2004), mais ce composé présente de nombreux effets indésirables.

Face aux inconvénients découlant de l'utilisation de ces inhibiteurs, il est particulièrement intéressant pour l'industrie cosmétique de disposer d'agents cosmétiques utilisables dans le domaine de l'anti-âge, qui soient à la fois efficaces sur la cible visée, mais aussi suffisamment spécifiques pour limiter les effets indésirables consécutifs à une utilisation dans des compositions destinées à être appliquées sur la peau.

Ceci est d'autant plus important qu'une peau fragilisée, telle qu'une peau âgée se révèle particulièrement sensible et susceptible de réagir négativement à l'application desdites compositions.

Le but de la présente invention est de résoudre les problèmes et inconvénients mentionnés ci-dessus des techniques de l'art antérieur et de proposer une solution particulièrement avantageuse pour réguler la dégradation de constituants de la matrice extra-cellulaire, de façon à contrôler efficacement le remodelage cutané, prévenir ou atténuer les changements d'aspect et des propriétés de la peau liés au vieillissement, en particulier ceux liés aux agressions externes.

On connaît de **Gu et al.** (2005), l'utilisation d'un inhibiteur pharmacologique de l'enzyme MMP-9 (SB-3CT) pour traiter des désordres neurodégénératifs, mais il n'est pas fait mention d'aptamères. La demande WO 2010/110914 A2 suggère l'utilisation d'un inhibiteur de MMP9, par exemple un aptamère, pour inhiber une infection à HIV.

**Gatto et al.** (2009) et **Tucker et al.** (2012) décrivent les caractéristiques générales des aptamères à structure G-quadruplexe.

La demande WO 2013/153138 et l'article de **Da Rochas Gomes et al.,** (2012) décrivent des aptamères qui lient spécifiquement à la protéine MMP-9, et permettent d'identifier des tumeurs par imagerie médicale.

Mais aucun de ces documents de l'art antérieur ne décrit un aptamère d'ADN selon l'invention ni son utilisation pour des applications cutanées.

Résumé de l'invention

Les objets de l'invention sont définis dans les revendications 1 à 11.

Résumé de la description

La description porte sur un inhibiteur de la métalloprotéase-9 (MMP-9), utilisable par voie topique pour réguler la dégradation de constituants de la matrice extra-cellulaire et ainsi contrôler le remodelage cutané et/ou prévenir l'apparition de signes du vieillissement cutané ou en atténuer l'évolution.

Les présents inventeurs ont observé de façon surprenante qu'un aptamère d'ADN capable de se lier spécifiquement à la protéine MMP-9, et d'inhiber toute activité enzymatique de ladite protéine, remplissait tous ces critères. En particulier, cet aptamère, qui présente une structure en G-quadruplexe, est capable de pénétrer dans les cellules de la peau.

Pour sélectionner et isoler des aptamères inhibiteurs de la MMP-9, les inventeurs ont utilisé une banque de séquences oligonucléotidiques phosphotriester (même composition que l'ADN naturel). La sélection a été faite de façon dirigée pour identifier, isoler et caractériser des séquences pouvant interagir spécifiquement avec la metalloprotéase-9 et en inhiber l'activité enzymatique sur la matrice extracellulaire.

Ces aptamères ont ensuite été testés pour leur capacité à inhiber l'activité enzymatique de la MMP-9, sur substrat synthétique, sur cellules et sur un modèle de peau.

Un premier objet de la description concerne donc un aptamère à structure G-quadruplexe, capable d'inhiber l'activité enzymatique de la protéine MMP-9.

Un deuxième objet de la description concerne l'utilisation d'un aptamère selon l'invention pour inhiber l'activité enzymatique de la protéine MMP-9.

Un troisième objet de la description concerne une composition cosmétique ou pharmaceutique comprenant en tant qu'agent actif un aptamère selon l'invention en une quantité suffisante pour inhiber l'activité enzymatique de la protéine MMP-9 et un ou plusieurs excipients cosmétiquement ou pharmaceutiquement acceptables.

Un quatrième objet de la description concerne l'utilisation cosmétique d'un aptamère selon l'invention.

Un cinquième objet de la description concerne un aptamère selon l'invention en tant que médicament.

Un sixième objet de la description concerne un aptamère selon l'invention pour son utilisation dans le traitement et/ou la prévention de pathologies associées à une surexpression ou à une hyperactivité de la MMP-9.

Un septième objet de la description concerne un procédé de sélection d'un aptamère selon l'invention comprenant les étapes suivantes :

- sélection dans une banque d'oligonucléotides d'aptamères par la méthode SELEX contre le site catalytique de la protéine MMP-9,
- évaluation du potentiel à inhiber l'activité enzymatique de la protéine MMP-9 des aptamères sélectionnés à l'étape précédente,
- clonage et séquençage des aptamères ainsi sélectionnés.

**Résumé des figures**

**Figure 1 : séquences des aptamères d'ADN sélectionnés contre le domaine catalytique de la MMP-9**
Les séquences d'aptamères les plus représentées ont été rassemblées en groupes de séquences selon un motif consensus contenu dans les régions aléatoires qui est représenté par les nucléotides encadrés. Les régions communes (i.e. régions fixes) correspondant aux amorces ne sont pas représentées. Les groupes de séquences et les candidats (i.e. référence des aptamères) obtenus aux tours 8 et 11 sont indiqués à gauche, tandis que le nombre de séquences appartenant au même groupe est indiqué à droite. Les nucléotides indiqués en grisé sont susceptibles de contribuer aux quartets de G.

**Figure 2 : séquences des aptamères d'ADN raccourcis**
Les noms des séquences sont indiqués à gauche. Les séquences ont été tronquées afin de définir la région minimale nécessaire à l'interaction avec le domaine catalytique de la protéine MMP-9.

**Figure 3 : analyse d'homologie de séquences - quartets de G**
Des doublets de G des séquences 11F46, 8F27, 8F14, 8F11 et 8F21 pouvant être impliqués dans la formation de quartets de G sont indiqués en grisé. Des expériences de dichroïsme circulaire et de Tm ont confirmé que les séquences 11F46, 8F27 et 8F14 se structurent en quartets de G.

**Figure 4 : activité MMP-9**

Ce graphique représente l'inhibition de l'activité enzymatique de la MMP-9 des différents aptamères sélectionnés par rapport au maximum d'activité représenté par un témoin MMP-9 (barre noire).

**Figures 5 et 6 : effet dose sur l'activité MMP-9**

Ces graphiques représentent la capacité d'inhibition de l'activité enzymatique de la métalloprotéase MMP-9 de différents aptamères identifiés suivant la dose utilisée. La dose est indiquée en abscisses après le nom de l'aptamère.

## Description détaillée

Un premier exemple de la description concerne donc un aptamère à structure G-quadruplexe, capable d'inhiber l'activité enzymatique de la métalloprotéase MMP-9.

On entend par « aptamère » une molécule d'ADN ou d'ARN ligand spécifique et de forte affinité d'une protéine. Cette acception comprend par conséquent les aptamères « naturels » et les analogues modifiés chimiquement.

Par « structure G-quadruplexe », « G-quartet » ou « tétrade de G » selon l'invention, on entend une structure secondaire comprenant 4 guanines associées dans un plan cyclique via des appariements de type Hoogsteen. Dans cette structure, chaque guanine est impliquée dans quatre liaisons hydrogènes par l'intermédiaire des atomes N1, N7, O6 et N2 (**Williamson** et al., 1989 ; **Sundquist et Klug,** 1989 ; **Tucker** et al., 2012).

Par « activité enzymatique » relative à la protéine MMP-9, on entend une activité protéolytique conduisant à la dégradation des substrats matriciels ou bioactifs de cette protéine, tels que la gélatine qui est une forme dénaturée de collagène, l'aggrécane, l'entactine, l'élastine, les collagènes II, III, IV, V,XIV et XVII, la myéline, l'endostatine, le plasminogène, les inhibiteurs de sérine protéase, la substance P, les protéines CBP30 et CBP35, le récepteur alpha à l'interleukine 2 (IL2), l'inhibiteur de la voie du facteur tissulaire (TFPI), le peptide amyloïde béta, le pro-TNF alpha ou encore le -TGF béta, et les chimiokines CXC (**Van den Steen** et al., 2002; **Folgueras** et al., 2004; **Chaussain-Miller** et al., 2006). L'activité enzymatique de la protéine MMP-9 selon la présente invention est préférablement une activité gélatinase. Dans le cadre de la présente invention, la mesure de l'activité protéolytique de la MMP-9 peut être vérifiée selon la méthode décrite ci-après dans l'Exemple 3 : la mesure de l'activité enzymatique des MMPs y est basée sur le principe du transfert d'énergie par résonance, RET ou encore transfert d'énergie de fluorescence par résonance, FRET. Le substrat est constitué d'un oligopeptide comportant un groupement fluorescent (F), donneur d'énergie et un groupement éteignant (Q, pour quenching), accepteur d'énergie. Après hydrolyse, le groupement éteignant est libéré, permettant de mesurer l'augmentation de fluorescence. De nombreux couples fluorescent/éteignant ont été développés pour la mesure de l'activité enzymatique des MMPs dont le couple 7-méthoxycoumarine-4-acétyl (Mca)/dinitrophenyl-diaminopropionyl (Dnp) (**Knight** et al., 1991; **Knight** et al., 1992).

Les aptamères sont sélectionnés par alternance sélection/amplification qui permet de diriger l'évolution de la population selon un mode darwinien : dans la population, les molécules les plus aptes sont sélectionnés, d'où le nom « aptamères » donné aux oligonucléotides présentant le caractère désiré, issus de la sélection. Les techniques classiques de génie génétique (clonage, séquençage, expression) permettent d'identifier individuellement ces aptamères, de les caractériser et de les produire ensuite en quantité.

La sélection des aptamères peut être réalisée par un protocole optimisé de sélection *in vitro,* connu sous le nom de « SELEX » (« Systemic Evolution of Ligands by Exponential enrichment »), décrit notamment dans la demande internationale WO 91/19813.

Le procédé SELEX permet de générer des ligands de très haute affinité et spécificité en grande quantité. Cette approche repose sur la mise en contact de la molécule cible avec une banque de ligands potentiels. Un système de cycles de désorption/sélection permet d'enrichir la population des ligands interagissant le plus spécifiquement avec la molécule cible. La population obtenue en final est ensuite isolée et caractérisée permettant sa resynthèse à grande échelle.

Bien que le procédé SELEX ait été établi comme une technique générale pour sélectionner des aptamères, il n'est néanmoins ni prévisible ni standardisé pour n'importe quelle cible. Au contraire, le procédé SELEX doit être optimisé et adapté pour chaque cible particulière. Le procédé SELEX n'est pas garanti pour toute cible.

Plusieurs facteurs sont importants pour une sélection d'aptamères. Par exemple, la molécule cible doit être stable et facilement reproductible à chaque cycle de SELEX, parce que le procédé SELEX implique plusieurs cycles de liaison, sélection, et amplification. En outre, les acides nucléiques qui montrent une liaison spécifique à la cible doivent être présents dans la banque initiale. Ainsi, il est nécessaire de produire une banque initiale d'acides nucléiques très diversifié.

Compte tenu de ces facteurs critiques, la sélection d'aptamères en utilisant le procédé SELEX n'est ni prévisible, ni évidente. Même si tous les facteurs sont optimums pour la sélection d'aptamères, le procédé SELEX ne permet pas toujours d'obtenir des aptamères viables pour chaque cible.

La banque initiale de candidats est composée de séquences d'oligonucléotides synthétisées chimiquement, chacune comportant une région variable longue de n nucléotides flanquée, en 3' et en 5', de régions fixes identiques pour tous les candidats de la banque. Ces régions fixes permettent la manipulation de la partie centrale au cours du SELEX, notamment l'amplification par PCR. La taille de la portion variable dicte la diversité de la banque qui est égale à $4^n$ puisque chaque position peut être occupée par l'un des quatre nucléotides A, T (ou U), G ou C. Pour des fenêtres de grande taille, on atteint des complexités gigantesques : pour *n* = 50 la diversité théorique est de $4^{50}$ soit $10^{30}$, une valeur inaccessible en pratique puisqu'elle correspond à plus de $10^5$ tonnes pour une banque dans laquelle chaque séquence est représentée une fois. La limite expérimentale se situe autour de $10^{15}$ séquences différentes, soit celle d'une banque dans laquelle tous les candidats possédant une région variable de 25 nucléotides sont représentés. Si l'on choisit de manipuler une banque comportant une fenêtre de 30 nucléotides dont la diversité théorique est d'environ $10^{18}$, on n'explorera donc que 1 /1000$^e$ des possibilités.

D'ailleurs, les polymérases utilisées étant infidèles et introduisant des erreurs à un taux de l'ordre de $10^{-4}$, elles contribuent à enrichir significativement la diversité du pool de séquences tout au long du processus SELEX : un candidat sur 100 sera modifié à chaque cycle d'amplification pour une banque avec une région aléatoire de 100 nucléotides, conduisant donc à l'apparition de $10^{13}$ nouveaux candidats pour la banque totale.

La sélection s'opère à chaque tour, par séparation physique entre molécules associées à la cible et molécules libres. De multiples techniques peuvent être mises en oeuvre (chromatographie, rétention sur filtre, électrophorèse...). Les conditions de la sélection sont ajustées (concentration relative cible/candidats, concentration ionique, température, lavage...) pour qu'une compétition intervienne entre les candidats pour fixation à la cible. D'une façon générale, la stringence est augmentée au fil des tours de façon à favoriser la capture des candidats les plus affins. Par ailleurs, une contre-sélection est effectuée pour éliminer les candidats qui reconnaissent le support (filtre, billes...).

Les oligonucléotides sont des oligoanions, chaque motif unitaire possédant une charge à pH neutre, des sites donneurs/accepteurs de liaison hydrogène et un hétérocycle aromatique (la base nucléique) pouvant générer des interactions d'empilement. Ces oligomères se replient suite à la formation de paires de bases, pour générer des structures secondaires et tertiaires telles que les structures tige-boucle (ou épingle à cheveux), pseudo-noeud, ou G-quadruplexe. La banque de séquences initiales est donc une banque de formes tridimensionnelles, chacune correspondant à une distribution de motifs, susceptibles d'engager des interactions électrostatiques, de donner naissance à des liaisons H, etc. La sélection revient à identifier dans la banque la forme adaptée à la cible, c'est-à-dire celle permettant le plus grand nombre d'interactions, la formation du complexe aptamère-cible, le plus stable. Pour des cibles de petite taille (colorants, antibiotiques...), les aptamères identifiés sont caractérisés par des constantes d'équilibre de dissociation de l'ordre du micromolaire tandis que pour des cibles protéiques les $K_d$ inférieures à $10^{-9}$ M ne sont pas rares.

La propriété la plus remarquable des aptamères est la spécificité des interactions engagées avec leur ligand, ce qui en fait des agents de premier plan pour la reconnaissance d'une cible.

Ainsi, de préférence, l'aptamère selon l'invention se lie spécifiquement à la protéine MMP-9. On entend par « liaison spécifique » une interaction spécifique de l'aptamère avec sa cible, excluant toute interaction avec une cible étrangère qui présente une structure différente.

De manière encore préférée, ledit aptamère se lie spécifiquement avec une forte affinité à ladite protéine MMP-9. On entend par « liaison spécifique avec forte affinité » au sens de la présente invention une interaction spécifique de l'aptamère avec sa cible, avec une constante de dissociation ($K_d$) suffisamment faible pour permettre l'inhibition significative de l'activité catalytique de l'enzyme ciblée.

Dans le cadre de la présente invention, le site catalytique isolé de la protéine MMP-9 a été utilisé pour générer l'aptamère selon l'invention. L'homme du métier est à même d'isoler et d'exprimer ledit site catalytique à l'aide de techniques de biologie moléculaire bien établies dans l'art. De manière préférée, le fragment peptidique Phe88-Pro438 de la protéine MMP-9 humaine, qui contient le site catalytique de cette enzyme, a été utilisé pour générer l'aptamère selon l'invention (numéro d'accès de la protéine MMP-9 humaine : P14780 selon la base de données UniProtKB/Swiss-Prot).

Les aptamères de la description peuvent être des oligodésoxy-(ADN) ou des oligoribo-nucléotides (ARN). Dans ce dernier cas, la première étape du SELEX peut consister en une transcription d'une banque initiale d'ADN, la partie fixe 5' des candidats comportant une séquence promotrice. Après sélection, les candidats sont convertis en ADN par transcription inverse avant d'être amplifiés. Des aptamères ARN et ADN ayant des caractéristiques comparables ont été sélectionnés contre une même cible. De plus les deux composés sont inhibiteurs compétitifs l'un de l'autre suggérant un recouvrement des sites d'interaction. Cela a des conséquences majeures pour la réalisation d'aptamères chimiquement modifiés.

Le développement de l'approche antisens a conduit à la synthèse de très nombreux analogues dont certains par exemple confèrent à l'oligomère une résistance aux nucléases, une propriété utile pour une utilisation dans un environnement biologique (milieu de culture cellulaire ou *in vivo*). Des modifications de la liaison phosphodiester, du sucre ou du squelette phosphate-sucre tels les dérivés 2'-O-méthyle, acide nucléique « *locked* » ou borano-phosphate conduisent à des oligomères résistants aux nucléases. Cette propriété peut présenter un intérêt pour les aptamères. Cependant, comme mentionné précédemment, le changement complet de structure chimique *a posteriori* d'un aptamère sélectionné sous forme ARN ou ADN conduit généralement à une diminution voire à une perte des propriétés pour lesquelles il a été sélectionné. Cela ne signifie pas qu'il ne soit pas possible d'introduire des modifications à certaines positions. Mais il convient d'identifier les positions auxquelles les modifications sont tolérées. Cela peut être réalisé par essai de variants ponctuels ou par une approche systématique dite d'interférence chimique qui est une variante de la cartographie par empreinte.

De manière préférée, l'aptamère selon l'invention est un aptamère d'ADN.

Dans le cadre de l'invention, on préfère opérer directement la sélection d'oligonucléotides non naturels. Cela suppose que les nucléosides triphosphates modifiés soient efficacement incorporés et les matrices modifiées correctement lues par les polymérases utilisées au cours du SELEX. Un très petit nombre d'analogues satisfont aux exigences. Pour ce qui est des dérivés qui confèrent une résistance aux nucléases, les possibilités se limitent aux analogues phosphorothioate, boranophosphate, 2'-méthyle, 2'amino- ou 2'fluoro-pyrimidine, ces derniers étant de loin les plus utilisés. Les aptamères identifiés dans ce cas ont des nucléosides pyrimidiques modifiés et des résidus puriques non modifiés (2'hydroxyle). Ces molécules présentent une résistance accrue aux nucléases. Si nécessaire, des résidus puriques modifiés peuvent être introduits *a posteriori,* comme indiqué précédemment. Par ailleurs, il est possible de sélectionner des oligonucléotides comportant des substituants sur la position C(5) des pyrimidines ou N(7), C(8) des purines. Cela n'a pas d'effet sur la sensibilité aux nucléases, mais permet d'ajouter de nouvelles fonctionnalités (hydrophobicité, photoréactivité...). Une approche très différente concerne l'utilisation d'isomères optiques. Les acides nucléiques naturels sont les isomères D. Les analogues L sont résistants aux nucléases mais ne peuvent être produits par les polymérases. Selon les lois de l'isomérie optique, un aptamère en série L formera avec sa cible C un complexe ayant les mêmes caractéristiques que le complexe formé par l'isomère en série D et l'énantiomère C' de la cible C. Dès lors, si l'on peut synthétiser chimiquement le composé C', on l'utilisera pour réaliser la sélection d'un aptamère naturel (D). Une fois identifié, cet aptamère sera synthétisé chimiquement en série L. Cet aptamère L sera un ligand de la cible naturelle C.

Une autre approche, récemment décrite comme SELEX bidimensionnel, fait intervenir simultanément la sélection *in vitro* d'oligonucléotides et la chimie combinatoire dynamique (CCD), c'est-à-dire la mise en oeuvre d'une réaction réversible entre certains groupes de l'oligonucléotide (des groupements amines) et une banque de composés aldéhydiques. La réaction aboutit à la production d'oligonucléotides imines qui sont sélectionnés sur les mêmes principes que pour le SELEX classique. Il a ainsi été possible d'identifier pour une cible ARN en épingle des aptamères modifiés qui diffèrent des aptamères naturels.

Au contraire des modifications de squelette qui peuvent altérer la structure et qui nécessitent que des précautions soient prises avant d'être introduites sous peine de perdre les propriétés d'interaction de l'aptamère avec sa cible, il est possible de conjuguer différents groupements à l'une des extrémités 3' ou 5' de l'oligonucléotide pour le convertir en outil, sonde ou capteur sans perturber ses caractéristiques. Cette versatilité constitue d'ailleurs un intérêt important des aptamères, notamment dans des perspectives diagnostiques.

L'expression « analogue d'aptamère » s'entend ici de l'une ou plusieurs des modifications décrites ci-dessus.

Selon un mode préféré de l'invention, l'aptamère est résistant aux nucléases. Préférentiellement, l'aptamère selon l'invention comprend au moins une modification de la liaison phosphodiester, de la partie sucre ou du squelette phosphate-sucre choisie dans le groupe des dérivés 2'-alkyle, 2'-amino et 2'-fluoro sur la partie sucre, des dérivés phosphorothioates, méthylphosphonates ou boranophosphates au niveau du squelette, ou des LNA (« Locked Nucleic Acids ») ou PNA (« Peptide Nucleic Acids »).

Selon des exemples de la description, l'aptamère comprend une séquence nucléotidique choisie dans le groupe consistant en les séquences SEQ ID NO :1 à SEQ ID NO :10, telles que définies ci-dessous :

- 5'$X_1X_2X_3X_4$TTTGGTGGGTYTGGGGTWGYK$X_5X_6$3', où X représente 0 ou 1 nucléotide G (SEQ ID NO :1) objet de l'invention ;
- 5'TGGCRCGGGGTTGGTGTYGGGTT3' (SEQ ID NO :2) ;
- 5'GGGWTTGGCTT$X_7$CGGYGCCTGGCG3', où X représente 0 ou 1 nucléotide A (SEQ ID NO :3);
- 5'GTGGTTGG$X_8$GSKRTRGWKGKT3', où X représente 0 ou 1 nucléotide T (SEQ ID NO :4);
- 5'GGGTGGGGGGGTGG3' (SEQ ID NO :5);
- 5'TTGGTGGGATGGGGGGGGGTTGTTCGGCT3' (SEQ ID NO :6);
- 5'CTGGGGGTGTGTYGCGATTGTGTGGGTGGG3' (SEQ ID NO :7);
- 5'SCSCGGTGGAYTGGTTGGGTTTGGATCCCC3' (SEQ ID NO :8);
- 5'TGAGGGGGGTGGATGGGAGGGTTCCGCACG3' (SEQ ID NO :9); et
- 5'TGGACGGTGGGTTGGGGCGGGGGGTGTCCA3' (SEQ ID NO :10).

Selon un mode de réalisation, l'aptamère selon l'invention comprend une séquence nucléotidique consistant en la séquence consensus 5'$X_1X_2X_3X_4$TTTGGTGGGTYTGGGGTWGYK$X_5X_6$3', où X représente 0 ou 1 nucléotide G (SEQ ID NO :1) ou l'une des séquences SEQ ID NO :11 à SEQ ID NO :17, SEQ ID NO : 32 à SEQ ID NO : 34, et SEQ ID NO :40 à SEQ ID NO : 45 comprises dans la séquence consensus SEQ ID NO :1.

Lesdites séquences sont des séquences consensus conformes à la nomenclature IUPAC communément utilisée pour désigner les nucléotides (Tableau 1).

**Tableau 1**

| code à une lettre des nucléotides | nucléotides correspondants |
|---|---|
| A | adénine |
| G | guanine |
| C | cytosine |
| T | thymine |
| U | uracile |
| R | adénine ou guanine (purines) |
| Y | cytosine ou thymine (pyrimidine) |
| N | n'importe quel nucléotide |
| W | adénine ou thymine (faible) |
| S | guanine ou cytosine (fort) |
| M | adénine ou cytosine (amino) |
| K | guanine ou thymine (céto) |
| B | pas l'adénine (guanine, cytosine ou thymine) |
| H | pas la guanine (adénine, cytosine ou thymine) |
| D | pas la cytosine (adénine, guanine ou thymine) |
| V | pas la thymine (adénine, guanine ou cytosine) |

L'aptamère selon un exemple de la description comprend une séquence nucléotidique choisie dans le groupe consistant en les séquences SEQ ID NO :6 à SEQ ID NO :29.

Selon mode particulier de l'invention, ladite séquence nucléotidique comprend au moins 1 à 24 nucléotides contigus de la séquence SEQ ID NO :30 flanquée à son extrémité 5', et/ou au moins 1 à 23 nucléotides contigus de la séquence SEQ ID NO :31 flanquée à son extrémité 3'.

De manière encore préférée, ladite séquence nucléotidique selon la description est choisie dans le groupe consistant en les séquences :

SEQ ID NO :6 à SEQ ID NO :10, SEQ ID NO :12 à SEQ ID NO :17, SEQ ID NO :19 à SEQ ID NO :20, SEQ ID NO :22 à SEQ ID NO :25, et SEQ ID NO :27 à SEQ ID NO :29,

et comprend au moins 1 à 24 nucléotides contigus de la séquence SEQ ID NO :30 flanquée à son extrémité 5', et/ou au moins 1 à 23 nucléotides contigus de la séquence SEQ ID NO :31 flanquée à son extrémité 3'.

On peut ainsi citer par exemple dans la description les séquences SEQ ID NO :32 à SEQ ID NO :39, et de manière préférée les séquences SEQ ID NO :40 à SEQ ID NO :59.

Les séquences particulièrement préférées selon la description sont sélectionnées parmi les séquences SEQ ID NO :12, SEQ ID NO :32 à SEQ ID NO :40, SEQ ID NO :46, et SEQ ID NO :48. Les séquences particulièrement préférées selon l'invention sont sélectionnées parmi les séquences SEQ ID NO :12, SEQ ID NO :32 et SEQ ID NO :40.

Le Tableau 2 suivant résume ces séquences de la description qui ont été rassemblées en 10 principaux groupes de séquences présentant une forte homologie (i.e. groupes I à X). L'homme du métier comprendra aisément du Tableau 2 ci-après que :

- les séquences SEQ ID NO :11 à 17, 32 à 34, et 40 à 45 sont comprises dans la séquence consensus SEQ ID NO :1 (groupe I), objets de l'invention;
- les séquences SEQ ID NO :18 à 20, 35 à 37, et 46 et 47 sont comprises dans la séquence consensus SEQ ID NO :2 (groupe II), exemples de la description ;
- les séquences SEQ ID NO :21 à 23, 38, 39, 48 et 49 sont comprises dans la séquence consensus SEQ ID NO :3 (groupe III) exemples de la description ;
- les séquences SEQ ID NO :24 à 27, et 50 à 52 sont comprises dans la séquence consensus SEQ ID NO :4 (groupe IV) exemples de la description ;
- les séquences SEQ ID NO :28, 29, 53 et 54 sont comprises dans la séquence consensus SEQ ID NO :5 (groupe V) exemples de la description ;

- la séquence SEQ ID NO :55 est comprise dans la séquence consensus SEQ ID NO :6 (groupe VI) exemples de la description ;
- la séquence SEQ ID NO :56 est comprise dans la séquence consensus SEQ ID NO :7 (groupe VII) exemples de la description ;
- la séquence SEQ ID NO :57 est comprise dans la séquence consensus SEQ ID NO :8 (groupe VIII) exemples de la description ;
- la séquence SEQ ID NO :58 est comprise dans la séquence consensus SEQ ID NO :9 (groupe IX) exemples de la description ; et
- la séquence SEQ ID NO :59 est comprise dans la séquence consensus SEQ ID NO :10 (groupe X) exemples de la description

**Tableau 2**

| SEQ ID NO | Séquence nucléotidique (5' à 3') | Référence |
|---|---|---|
| SEQ ID NO:30 | GCCTGTTGTGAGCCTCCTGTCGAA | région fixe 5' |
| SEQ ID NO:31 | TTGAGCGTTTATTCTTGTCTCCC | région fixe 3' |
| **Groupe I** | | |
| SEQ ID NO:1 | $X_1X_2X_3X_4$TTTGGTGGGTYTGGGGTWGYK$X_5X_6$ | consensus I |
| SEQ ID NO:11 | TTTGGTGGGTCTGGGGTTGCT | 11F46min |
| SEQ ID NO:12 | TGGGGTTTGGTGGGTCTGGGGTTGCTGGCC | 11F46R |
| SEQ ID NO:32 | TCGAATGGGGTTTGGTGGGTCTGGGGTTGCTGGCCTTGAGC | 11F46A |
| SEQ ID NO:33 | TCGAATGGGGTTTGGTGGGTCTGGGGTTGCT | 11F46B |
| SEQ ID NO:34 | TTTGGTGGGTCTGGGGTTGCTGGCCTTGAGC | 11F46C |
| SEQ ID NO:40 | GCCTGTTGTGAGCCTCCTGTCGAATGGGGTTTGGTGGGTCTGGGGTTGCTGGCCTTGAGCGTTTATTCTTGTCTCCC | 11F46 |
| SEQ ID NO:13 | TGGGGTTTGGTGGGTTTGGGGTTGCTGGCC | 11F76R |
| SEQ ID NO:41 | GCCTGTTGTGAGCCTCCTGTCGAATGGGGTTTGGTGGGTTTGGGGTTGCTGGCCTTGAGCGTTTATTCTTGTCTCCC | 11F76 |
| SEQ ID NO:14 | TAGGGTTTGGTGGGTCTGGGGTTGCTGGCC | 11F89R |
| SEQ ID NO:42 | GCCTGTTGTGAGCCTCCTGTCGAATAGGGTTTGGTGGGTCTGGGGTTGCTGGCCTTGAGCGTTTATTCTTGTCTCCC | 11F89 |
| SEQ ID NO:15 | CCGGGGTTTGGTGGGTCTGGGGTAGTTGGC | 11F57R |
| SEQ ID NO:43 | GCCTGTTGTGAGCCTCCTGTCGAACCGGGGTTTGGTGGGTCTGGGGTAGTTGGCTTGAGCGTTTATTCTTGTCTCCC | 11F57 |
| SEQ ID NO:16 | TTGGGGTTTGGTGGGTCTGGGGTTGCGGGT | 8F68R |
| SEQ ID NO:44 | GCCTGTTGTGAGCCTCCTGTCGAATTGGGGTTTGGTGGGTCTGGGGTTGCGGGTTTGAGCGTTTATTCTTGTCTCCC | 8F68 |
| SEQ ID NO:17 | GCGGGGTTTGGTGGGTCTGGGGTTGTTGGT | 8F44R |
| SEQ ID NO:45 | GCCTGTTGTGAGCCTCCTGTCGAAGCGGGGTTTGGTGGGTCTGGGGTTGTTGGTTTGAGCGTTTATTCTTGTCTCCC<br>************* ****** * ** | 8F44 |
| **Groupe II** | | |
| SEQ ID NO:2 | TGGCRCGGGGTTGGTGTYGGGTT | consensus II |
| SEQ ID NO:18 | TATGGCACGGGGTTGGTGTTGGGTT | 8F14min |
| SEQ ID NO:19 | TCGTATGGCACGGGGTTGGTGTTGGGTTGG | 8F14R |
| SEQ ID NO:35 | TCGAATCGTATGGCACGGGGTTGGTGTTGGGTTGGTTGAGC | 8F14A |
| SEQ ID NO:36 | TCGAATCGTATGGCACGGGGTTGGTGTTGGGTT | 8F14B |
| SEQ ID NO:37 | TATGGCACGGGGTTGGTGTTGGGTTGGTTGAGC | 8F14C |
| SEQ ID NO:46 | GCCTGTTGTGAGCCTCCTGTCGAATCGTATGGCACGGGGTTGGTGTTGGGTTGGTTGAGCGTTTATTCTTGTCTCCC | 8F14 |
| SEQ ID NO:20 | CTGGCGCGGGGTTGGTGTCGGGTTTGGTTT | 8F19R |

(suite)

| | | |
|---|---|---|
| **Groupe II**<br>SEQ ID NO:47 | GCCTGTTGTGAGCCTCCTGTCGAACTGGCGCGGGGTTGGTGTCGGGTTTGGTTTTGAGCGTTTATTCTTGTCTCCC<br>**** ************ ***** | 8F19 |
| **SEQ ID NO** | **Séquence nucléotidique (5' à 3')** | **Référence** |
| **Groupe III** | | |
| SEQ ID NO:3 | GGGWTTGGCTTX$_7$CGGYGCCTGGCG | consensus III |
| SEQ ID NO:21 | CGAGGGTTTGGCTTACGGCGCCTGGCG | 8F27min |
| SEQ ID NO:22 | CCGCGAGGGTTTGGCTTACGGCGCCTGGCG | 8F27R |
| SEQ ID NO:38 | TCGAACCGCGAGGGTTTGGCTTACGGCGCCTGGCGTTGAGC | 8F27A |
| SEQ ID NO:39 | CGAGGGTTTGGCTTACGGCGCCTGGCGT | 8F27B |
| SEQ ID NO:48 | GCCTGTTGTGAGCCTCCTGTCGAACCGCGAGGGTTTGGCTTACGGCGCCTGGCGTTGAGCGTTTATTCTTGTCTCCC | 8F27 |
| SEQ ID NO:23 | CCGT-TGGGATTGGCTT-CGGTGCCTGGCGTG | 8F50R |
| SEQ ID NO:49 | GCCTGTTGTGAGCCTCCTGTCGAACCGT-TGGGATTGGCTT-CGGTGCCTGGCGTGTTGAGCGTTTATTCTTGTCTCCC<br>*** *** ******* *** ******** | 8F50 |
| **Groupe IV** | | |
| SEQ ID NO:4 | GTGGTTGGX$_8$GSKRTRGKKGKT | consensus IV |
| SEQ ID NO:24 | TGGTGGTTGGTGGGGTGGAGGTTAGGTACC | 8F11R |
| SEQ ID NO:50 | GCCTGTTGTGAGCCTCCTGTCGAATGGTGGTTGGTGGGGTGGAGGTTAGGTACCTTGAGCGTTTATTCTTGTCTCCC | 8F11 |
| SEQ ID NO:25 | MGTAGTGGTTGG-GCTGTAGTGGTT-GGGACC | 8F70R |
| SEQ ID NO:51 | GCCTGTTGTGAGCCTCCTGTCGAAMCTAGTGCTTGG-GCTGTAGTGGTT-GGGACCTTGAGCGTTTATTCTTGTCTCCC | 8F70 |
| SEQ ID NO:26 | GTGGTTGG-GGTATGGTTGGTACAGGTT | 8F77min |
| SEQ ID NO:27 | GAAGTGGTTGG-GGTATGGTTGGTACAGGTT | 8F77R |
| SEQ ID NO:52 | GCCTGTTGTGAGCCTCCTGTCGAAGAAGTGGTTGG-GGTATGGTTGGTACAGGTTTTGAGCGTTTATTCTTGTCTCCC<br>******** * * * * * | 8F77 |
| **Groupe V** | | |
| SEQ ID NO:5 | GGGTGGGGGGGTGG | consensus V |
| SEQ ID NO:28 | TGGCTGGYGACCTTGCGGGTGGGGGGGTGG | 8F21R |
| SEQ ID NO:29 | CCTGCGCCGTGATTAGGGGTGGGGGGGTGG | 8F67R |

(suite)

| | | |
|---|---|---|
| **Groupe V**<br>SEQ ID NO:53<br>SEQ ID NO:54 | GCCTGTTGTGAGCCTCCTGTOGAATGGCTGGYGACCTTGCGGGTGGGGGGGTGGTTGAGCGTTTATTCTTGTCTCCC<br>GCCTGTTGTGAGCCTCCTGTCGAACCTGCGCCGTGATTAGGGGTGGGGGGGTGGTTGAGCGTTTATTCTTGTCTCCC<br>* ** ************** | 8F21<br>8F67 |
| **Groupe VI**<br>SEQ ID NO:6<br>SEQ ID NO:55 | TTGGTGGGATGGGGGGGGGTTGTTCGGCT<br>GCCTGTTGTGAGCCTCCTGTCGAATTGGTGGGATGGGGGGGGGTTGTTCGGCTTTGAGCGTTTATTCTTGTCTCCC<br>****************************** | 8F5R<br>8F5 |
| **Groupe VII**<br>SEQ ID NO:7<br>SEQ ID NO:56 | CTGGGGGTGTGTYGCGATTGTGTGGGTGGG<br>GCCTGTTGTGAGCCTCCTGTCGAACTGGGGGTGTGTYGCGATTGTGTGGGTGGGTTGAGCGTTTATTCTTGTCTCCC<br>****************************** | 8F9R<br>8F9 |
| **Groupe VIII**<br>SEQ ID NO:8<br>SEQ ID NO:57 | SCSCGGTGGAYTGGTTGGGTTTGGATCCCC<br>GCCTGTTGTGAGCCTCCTGTCGAASCSCGGTGGAYTGGTTGGGTTTGGATCCCCTTGAGCGTTTATTCTTGTCTCCC<br>****************************** | 8F60R<br>8F60 |
| **Groupe IX**<br>SEQ ID NO:9<br>SEQ ID NO:58 | TGAGGGGGGTGGATGGGAGGGTTCCGCACG<br>GCCTGTTGTGAGCCTCCTGTCGAATGAGGGGGGTGGATGGGAGGGTTCCGCACGTTGAGCGTTTATTCTTGTCTCCC<br>****************************** | 8F65R<br>8F65 |
| **Groupe X**<br>SEQ ID NO:10<br>SEQ ID NO:59 | TGGACGGTGGGTTGGGGCGGGGGGTGTCCA<br>GCCTGTTGTGAGCCTCCTGTCGAATGGACGGTGGGTTGGGGCGGGGGGTGTCCATTGAGCGTTTATTCTTGTCTCCC<br>****************************** | 11F2R<br>11F2 |

Un autre objet de l'invention concerne l'utilisation d'un aptamère selon l'invention, pour inhiber l'activité enzymatique de la protéine MMP-9.

L'activité enzymatique peut être mesurée selon la méthode décrite ci-dessus, notamment selon la méthode de l'Exemple 3 ci-après.

Un autre objet de l'invention concerne une composition cosmétique ou pharmaceutique comprenant en tant que principe actif au moins un aptamère selon l'invention en une quantité suffisante pour inhiber l'activité enzymatique de la protéine MMP-9 et un ou plusieurs excipients cosmétiquement/pharmaceutiquement acceptables.

De préférence, la composition de l'invention comprend de 0,000001% à 10%, de préférence de 0,000002% à 5 %, de manière encore préférée de 0.000005 à 1% en poids de la composition d'un ou plusieurs aptamères selon l'invention.

D'une manière générale, toute composition de l'invention peut être appliquée sur la peau.

Elle peut se présenter sous toutes les formes galéniques normalement utilisées pour une application topique sur la peau.

La composition de l'invention peut avoir la forme notamment de solutions aqueuses ou huileuses ou de dispersions du type lotion ou sérum, d'émulsions de consistance liquide ou semi-liquide du type lait, obtenues par dispersion d'une phase aqueuse dans une phase siliconée (E/Si), d'une phase grasse dans une phase aqueuse (H/E : émulsion huile-dans-eau) ou inversement (E/H : émulsion eau-dans-huile), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles. Les quantités des différents constituants des compositions selon l'invention sont celles classiquement utilisées dans les domaines considérés.

Dans le domaine de la cosmétique, ces compositions constituent notamment des crèmes de nettoyage, de protection, de traitement ou de soin pour le visage, pour les mains, pour les pieds, ou pour le corps (par exemple crèmes de jour, crèmes de nuit, crèmes de démaquillage, crèmes de fond de teint, crèmes antisolaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires.

Les compositions selon l'invention peuvent également consister en des préparations solides pulvérulentes ou non, par exemple sous forme de stick, d'une poudre pressée, des savons ou des pains de nettoyage. Elle peut encore se présenter sous la forme de patchs, de crayons, de pinceaux et d'applicateurs autorisant une application localisée sur les taches du visage ou des mains. Elle peut être utilisée comme produit de soin ou comme produit de maquillage.

Lorsque la composition est une émulsion, la proportion de la phase grasse peut varier d'environ 5 % à 80 % en poids, et de préférence d'environ 5 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30% en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique ou pharmaceutique de l'invention peut contenir également des adjuvants habituels dans le domaine cosmétique ou pharmaceutique, tels que les gélifiants hydrophiles ou lipophiles, les additifs hydrophiles ou lipophiles, les conservateurs, les antioxydants, les solvants, les parfums, les charges, les filtres, les pigments, les absorbeurs d'odeurs et les matières colorantes. Les quantités de ces différents adjuvants sont celles classiquement utilisées dans les domaines considérés, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles de synthèse, les huiles ou cires siliconées (cyclométhicone), les cires d'abeille, de carnauba ou paraffine. On peut ajouter à ces huiles des alcools gras et des acides gras (acide stéarique). Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefosse 63 par la société Gattefosse.

Comme solvants utilisables dans l'invention, on peut citer les alcools inférieurs notamment l'éthanol et l'isopropanol et le propylène glycol.

Comme gélifiants hydrophiles utilisables dans l'invention, on peut citer les polymères carboxyvinyliques (carbomères), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates de magnésium, la silice hydrophobe, l'éthylcellulose et le polyéthylène.

Une composition de l'invention peut également comprendre un ou plusieurs autres principes actifs, par exemple destiné(s) à prévenir ou à lutter contre l'apparition de signes du vieillissement cutané (composition cosmétique), ou à prévenir et/ou traiter des pathologies associées à une surexpression et/ou à une hyperactivité de la MMP-9 (composition pharmaceutique). Les pathologies associées à une surexpression et/ou à une hyperactivité de la MMP-9 sont par exemple les maladies de la peau, préférentiellement sélectionnées parmi les maladies inflammatoires de la peau telles que le psoriasis, les tumeurs cutanées telles le carcinome basocellulaire, les lésions cutanées telles que les plaies chroniques, les pathologies de la cicatrisation, les dermatoses bulleuses telles que la pemphigoïde bulleuse, les maladies granulomateuses non infectieuses telles que la sarcoïdose de la peau, le granulome annulaire et la nécrobiose lipoïdique, et les pathologies cutanées liées à une exposition aux rayons solaires ultra-violets telles que le mélanome. D'autres exemples, non limitatifs, de pathologies associées à une surexpression et/ou à une hyperactivité de la MMP-9 sont les pathologies tumorales, l'asthme, l'emphysème pulmonaire, la silicose, la bronchectasie, le purpura anaphylactoïde, le syndrome de détresse respiratoire aiguë (SDRA), l'arthrite rhumatoïde, la périodontite, les maladies intestinales inflammatoires, le lupus néphrétique, le syndrome de Sjögren, l'artérite à cellules géantes, l'anévrisme, les traumatismes des nerfs périphériques, la maladie d'Alzheimer, le syndrome de Guillain-Barré, la fibrose kystique, et les méningites.

Lesdits actifs utilisables en association avec les aptamères selon l'invention pour prévenir ou lutter contre l'apparition des signes du vieillissement cutané, utilisés purs ou provenant d'extraits renfermant ces molécules, sont notamment les composés suivants, sans limitation : les filtres UV (physiques ou chimiques), le rétinol, les esters de rétinol tel que le propionate de rétinol ou le palmitate de rétinol, la bêta-ecdysone, les anti-oxydants ou anti-inflammatoires tels que l'acide ascorbique ou ses dérivés tels que l'ascorbyl-2-glucoside ou le 3-O-ethyl ascorbyl,les dérivés de tocophérol tels que les tocophéryl phosphate ou le potassium ascorbyl tocopheryl phosphate, le glycyrrhizinate dipotassique et l'asiaticoside.

La présente invention a également pour objet l'utilisation d'au moins un aptamère selon l'invention dans une composition cosmétique pour traiter ou prévenir l'apparition des signes, visibles ou non, du vieillissement cutané intrinsèque et/ou extrinsèque ou pour en ralentir ou atténuer les effets, en particulier pour contrôler le remodelage cutané, restructurer l'épiderme, raffermir la peau, et/ou prévenir ou favoriser l'atténuation ou la résorption des rides.

L'expression « vieillissement cutané » est ici considérée dans son acception la plus large. Elle est associée à au moins une condition choisie parmi la désintégration de la structure des faisceaux de fibres de collagène, la formation des rides, la disparition de l'élasticité de la peau, la modification de la texture de la peau et la réduction de la différence entre un sillon et une élévation de la surface de la peau.

Plus particulièrement, par « vieillissement intrinsèque », connu également sous le nom de vieillissement « normal » ou chrono-biologique, on entend ici les modifications physiologiques au niveau moléculaire, cellulaire et/ou tissulaire d'un sujet liées à une sénescence programmée où interviennent des facteurs endogènes. Ce vieillissement intrinsèque provoque notamment un ralentissement du renouvellement des cellules de la peau, les kératinocytes, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Par « vieillissement extrinsèque », on entend ici des modifications physiologiques au niveau moléculaire, cellulaire et/ou tissulaire d'un sujet liées à des stimulations externes telles que des stimulations chimiques et physiques excessives. Les stimulations chimiques et physiques pouvant détériorer les fonctions normales de la peau et induire son vieillissement incluent notamment une exposition au soleil, à la lumière, aux UV, le stress ainsi que la malnutrition. Ce vieillissement extrinsèque entraîne des altérations cliniques telles que des rides profondes et la formation d'une peau ayant perdu sa fermeté, sa souplesse et son élasticité. Ces transformations sont dues essentiellement à des changements histopathologiques, tels qu'une modification excessive du tissu élastique dans le derme supérieur et une dégénérescence quantitative et qualitative des fibres de collagène.

Par « remodelage cutané », ou restructuration cutanée, on entend l'action concertée d'enzymes de dégradation et de synthèse de la matrice extracellulaire des cellules de la peau, telles que les cellules de l'épiderme, du derme et/ou de l'hypoderme. En effet, les enzymes de dégradation de la matrice extracellulaire régulent à la fois la dégradation de la matrice extracellulaire mais aussi sa synthèse afin de créer un environnement approprié permettant la différenciation cellulaire, la prolifération ou la migration. L'aptamère selon l'invention permet ici de contrôler ce remodelage cutané en protégeant les matrices extracellulaires épidermiques et/ou dermiques et/ou hypodermiques.

La présente invention a aussi pour objet l'utilisation d'au moins un aptamère selon l'invention dans ou pour la fabrication d'une composition cosmétique ou pharmaceutique comme inhibiteur de la MMP-9.

La présente invention a aussi pour objet l'utilisation d'au moins un aptamère selon l'invention, dans une composition cosmétique anti-rides.

La présente invention a encore pour objet l'utilisation d'au moins un aptamère selon l'invention pour la fabrication d'une composition dermatologique anti-rides.

Un autre objet de l'invention concerne l'utilisation cosmétique d'un aptamère selon l'invention, de préférence pour traiter ou prévenir l'apparition des signes, visibles ou non, du vieillissement cutané intrinsèque et/ou extrinsèque ou pour en ralentir ou atténuer les effets, en particulier pour contrôler le remodelage cutané, restructurer l'épiderme, raffermir la peau, et/ou prévenir ou favoriser l'atténuation ou la résorption des rides.

En particulier, l'utilisation cosmétique selon l'invention a pour but d'atténuer les rides et ridules, notamment celles apparaissant sur le visage, le cou, le décolleté ou les mains.

La présente invention se rapporte également à un procédé de traitement cosmétique ou dermatologique pour traiter ou prévenir l'apparition des signes, visibles ou non, du vieillissement cutané intrinsèque et/ou extrinsèque ou pour en ralentir ou atténuer les effets, en particulier pour contrôler le remodelage cutané, restructurer l'épiderme, raffermir la peau, et/ou prévenir ou favoriser l'atténuation ou la résorption des rideset/ou limiter le développement du tissu adipeux, consistant à appliquer sur une zone de peau concernée du corps ou du visage une composition cosmétique ou dermatologique comprenant au moins un aptamère selon l'invention.

De préférence on applique la composition de l'invention une fois par jour sur la ou les zones de peau concernées. Avantageusement, on applique la composition une première fois et à nouveau le soir, sur les mêmes zones.

La présente invention concerne également l'utilisation d'au moins un aptamère selon l'invention pour la fabrication d'un médicament destiné à être administré de façon simultanée, séparée ou étalée dans le temps en association avec un ou plusieurs autres actifs, par exemple les actifs décrits ci-dessus.

La présente invention se rapporte également à l'utilisation d'au moins un aptamère selon l'invention pour la fabrication d'un médicament destiné au traitement et/ou à la prévention de pathologies associées à une surexpression et/ou à une hyperactivité de la MMP-9, lesdites pathologies étant de préférence sélectionnées parmi celles décrites ci-dessus. De manière encore préférée, les dites pathologies sont des maladies de la peau telles que celles décrites ci-dessus.

Un autre objet de l'invention concerne un aptamère selon l'invention en tant que médicament, de préférence destiné au traitement et/ou à la prévention de pathologies associées à une surexpression et/ou à une hyperactivité de la MMP-9, lesdites pathologies étant préférentiellement sélectionnées parmi celles décrites ci-dessus. De manière encore préférée, les dites pathologies sont des maladies de la peau telles que celles décrites ci-dessus.

Un autre objet de l'invention concerne un aptamère selon l'invention pour son utilisation dans le traitement et/ou la prévention pathologies associées à une surexpression et/ou à une hyperactivité de la MMP-9, lesdites pathologies étant de préférence sélectionnées parmi celles décrites ci-dessus. De manière encore préférée, les dites pathologies sont des maladies de la peau telles que celles décrites ci-dessus.

Les aptamères selon l'invention peuvent être utilisés sous forme vectorisée, c'est-à-dire liés à un vecteur, ou à une combinaison de vecteurs, notamment pour faciliter leur pénétration dans les cellules de la peau. Ce type de vecteur est bien connu de l'homme du métier. Des exemples de tels vecteurs incluent, sans limitation, les liposomes, notamment les liposomes cationiques, les résidus hydrophobiques tels que le cholestérol, les dendrimères, notamment les dendrimères polycationiques, les nanoparticules, les microencapsulations, les peptides pénétrant les cellules tels que les domaines de transduction peptidiques (PTD), les agents de condensation des acides nucléiques tels que la polyéthylène imine (PEI) ou la poly-L-lysine.

Les aptamères selon l'invention peuvent également être utilisés sous forme de dimère de deux aptamères ou d'un conjugué de plusieurs aptamères.

Les aptamères selon l'invention peuvent également être couplés à des principes actifs destiné(s) à prévenir ou à lutter contre le vieillissement cutané ou à traiter et/ou prévenir les maladies de la peau associées à une surexpression et/ou à une hyperactivité de la MMP-9, par exemples les principes actifs décrits ci-dessus.

Un autre objet de l'invention concerne un procédé de sélection d'un aptamère selon l'invention comprenant les étapes suivantes :

- sélection dans une banque d'oligonucléotides d'aptamères par la méthode SELEX contre le site catalytique de la protéine MMP-9,
- évaluation du potentiel à inhiber l'activité enzymatique de la protéine MMP-9 des aptamères identifiés à l'étape précédente,
- clonage et séquençage des aptamères ainsi sélectionnés.

Selon un mode de réalisation préféré de l'invention, ladite banque d'aptamères est une banque d'ADN.

La présente invention sera mieux comprise à la lumière des exemples ci-après. Néanmoins, l'homme du métier appréciera que la description ci-dessus n'est pas limitative et que diverses modifications, substitutions, omissions et des changements peuvent être apportés sans sortir du cadre de l'invention.

## EXEMPLES

### Exemple 1 : Sélection des aptamères anti-MMP9

#### 1.1. Protéines

Le domaine catalytique de la MMP-9 humaine recombinante contenant un tag 6-His en C-terminal a été obtenu de Biomol[r] international, les protéines MMP-9 et MMP-2 de Calbiochem.

#### 1.2. Oligonucléotides et banque

Une banque d'ADN et d'amorces fournies par Sigma ont été purifiés par HPLC. Les séquences amorces (P3) 5' GGGAGACAAGAATAAACGCTCAA 3' (SEQ ID NO :60) et (P5) 5' GCCTGTTGTGAGCCTCCTGTCGAA 3' (SEQ ID NO :30) ont été utilisés pour l'amplification de la banque contenant une fenêtre de 30 nucléotides au hasard. La séquence 5' ACTGACTGACTGACTGACTA-6C3-GGGAGACAAGAATAAACGCTCAA 3' (SEQ ID NO :61) a été utilisée pour produire du simple brin tel que décrit dans la littérature (Williams and **Bartel,** 1995). L'amorce (P3) biotinylée en 5' a été utilisée pour produire des candidats ADN simple brin. Des candidats ADN ont été synthétisés et purifiés par HPLC par Eurogentec (figures 1 et 2). Avant toute expérience, les populations et candidats ADN ont été chauffés à 75°C pendant 5 min, placés sur la glace 5 min, et ensuite mis à température ambiante pendant au moins 5 min.

#### 1.3. Sélection In vitro

Avant la sélection, la banque d'ADN (1 nanomole) a été traitée et incubée deux fois pour le premier et le second tours et une fois pour tous les autres tours en tampon SP (50 mM Tris-HCl, pH 7.4, 50 mM NaCl, 100 mM KCl, 5 mM $CaCl_2$, 1 mM $(CH_3COO)_2Mg$ avec des filtres (HAWP 0,45 μM, Millipore) pendant 20 min à température ambiante. A chaque tour, une contre-sélection supplémentaire a été réalisée contre l'étiquette 6-His-GST. Ensuite, la banque contre-sélectionnée a été mélangée au domaine catalytique de la MMP-9 (20 picomoles) pendant 20 min et les candidats non liés ont été séparés par la technique de rétention sur filtre. Après la filtration, les candidats liés au domaine catalytique de la MMP-9 ont été élués par incubation 20 min à 65°C dans 500 μL de phénol/urée 7M, précipités et amplifiés par PCR pour produire du simple brin utilisé pour les tours suivants de sélection. Réduire la quantité de candidats et de cible pendant la sélection pour atteindre 25 et 1 picomoles au onzième tour, respectivement, a augmenté la rigueur de la sélection. Avant le clonage, l'évolution des populations a été évaluée pour leur capacité à inhiber l'activité MMP-9.

#### 1.4. Clonage et séquençage

Après les 11 tours de sélection, les séquences sélectionnées des tours 8 et 11 ont été clonées en utilisant le kit de clonage TOPO TA (Invitrogen) et séquencées en utilisant le kit de séquençage BigDye Terminator v1.1 cycle (Applied Biosystems) selon les instructions du fabricant.

#### 1.5. Dénaturation thermique des aptamères d'ADN

Les aptamères d'ADN ont été préparés dans un tampon sodium cacodylate 20 mM, pH 7.3 à 20°C, contenant 140 mM de chlorure de potassium, 20 mM de chlorure de sodium et 3 mM de chlorure de magnésium. Les échantillons d'ADN ont été préparés à des concentrations finales de 3 μM ou 10 μM pour les candidats pleine longueur et raccourcis, respectivement. Les échantillons ont été dénaturés à 75°C pendant 5 min et places sur la glace pendant 5 min et incubés pendant 20 min à température ambiante. La dénaturation des échantillons a été réalisée par chauffage de 0,4°C/min de 4 à 90°C et a été suivie à 260 et 295 nm. La dénaturation thermique a été suivie dans un spectrophotomètre Uvikon interfacé avec un dispositif d'effet Peltier qui contrôle la température à ±0.1°C.

#### 1.6. Dichroïsme circulaire des aptamères d'ADN

Des spectres CD ont été réalisés sur un spectromètre de dichroïsme circulaire JASCO J-815 en utilisant des cellules de quartz de longueur de pas optique de 10-mm. Les balayages ont été réalisés à 23°C, avec un temps de réponse de 0,5s, une vitesse de balayage de 500 nm/min et sur une gamme de longueurs d'ondes de 230-320 nm. Une ligne de base pour la contribution au signal du tampon a été soustraite de chaque spectre.

Les oligonucléotides ont été préparés à 0,5 et 1 μM pour les aptamères pleine longueur et raccourcis, respectivement. Ils ont été chauffés à 70°C pendant 5 min dans l'eau, refroidis pendant 4 min à 4°C et conservés à température ambiante pendant 15 min dans le tampon cacodylate (sodium cacodylate 20 mM, KCl 140 mM, NaCl 20 mM, MgCl 3mM) jusqu'à l'analyse.

### 1.7. Résultats de la sélection des aptamères anti-MMP-9

La méthode SELEX a été utilisée contre le domaine catalytique de la MMP-9 pour identifier des aptamères d'ADN qui inhibent spécifiquement l'activité enzymatique. Onze tours de sélection *in vitro* ont été réalisés. Avant le clonage et le séquençage, les populations (en commençant par la banque au tour 11) ont été évaluées pour leur potentiel à inhiber l'activité MMP-9.

Sur la base de ces tests d'activité, les populations des tours 8 et 11 ont été clonées et séquencées.

La plupart des séquences possèdent des clusters de G qui peuvent donner des quartets de G. Les candidats ont été classés en cinq familles principales (I, II, III, IV, V), chaque famille contenant des séquences avec un motif consensus (voir les nucléotides encadrés de la Figure 1). Les autres séquences (8F5, 8F9, 8F60, 8F65 et 11F2) (groupe VI à X) n'ont pas de similarité avec les familles précédentes exceptée leur richesse en G.

## Exemple 2 : Mesure de l'activité MMP-9

### 2.1. Principe

Une enzyme est une protéine capable de catalyser spécifiquement la transformation d'un ou deux substrats. En prenant un modèle simplifié de réaction enzymatique : $S \xrightarrow{E} P$, la vitesse de réaction s'écrit : $v = \frac{d(P)}{dt} = -\frac{d(S)}{dt}$. Pour tracer une courbe (*P*) = *f*(*t*), l'enzyme E agit sur le substrat S ; le temps zéro correspond au déclenchement de la réaction. L'apparition du produit P est mesurée en fonction du temps.

La vitesse de réaction $v = \frac{d(P)}{dt}$ est constante pendant les conditions initiales. Pour cette portion de courbe, la tangente à l'origine se confond avec la courbe : la vitesse, pente de la tangente, est appelée vitesse initiale. Puis la vitesse diminue et s'annule. La vitesse s'annule lorsque l'un des substrats est consommé ou lorsqu'il s'établit un équilibre. Lorsque la vitesse d'une réaction enzymatique est déterminée, c'est toujours la vitesse initiale qui est calculée. Les mesures de vitesse sont donc faites dans les conditions initiales où moins de 10 % de la quantité de substrat est hydrolysé. Tant que [*S*] ϕϕ [*E*], la vitesse initiale est proportionnelle à la concentration de l'enzyme : elle traduit donc l'activité d'une préparation de l'enzyme exprimée en unités enzymatiques. L'unité internationale (UI ou U) représente la quantité d'enzyme qui catalyse la transformation d'une micromole de substrat par minute.

### 2.2. Mesure de l'activité enzymatique

La mesure de l'activité enzymatique des MMPs est basée sur le principe du transfert d'énergie par résonance, RET ou encore transfert d'énergie de fluorescence par résonance, FRET. Le substrat est constitué d'un oligopeptide comportant un groupement fluorescent (F), donneur d'énergie et un groupement éteignant (Q, pour quenching), accepteur d'énergie. Après hydrolyse, le groupement éteignant est libéré, permettant de mesurer l'augmentation de fluorescence.

De nombreux couples fluorescent/éteignant ont été développés pour la mesure de l'activité enzymatique des MMPs dont le couple 7-méthoxycoumarine-4-acétyl (Mca)/dinitrophenyl-diaminopropionyl (Dnp) (**Knight** et al., 1991).

Mesure de l'activité MMPs dans les milieux conditionnés

Après incubation des cellules ou modèles 3D en présence des différents candidats ou contrôles, les milieux conditionnés sont prélevés, centrifugés à 10 000 g pendant 10 min à +4°C afin d'éliminer les débris cellulaires et ajustés à la même concentration protéique. Le rouge de phénol contenu dans le milieu de culture est éliminé par diafiltration à l'aide de microconcentrateur Nanosep™ avec une membrane ayant un seuil de coupure de 10 kDa. 50 µl de milieux conditionnés sont centrifugés à 14 000 g pendant 6 min à +4°C. Le retentât est remis en suspension dans 50 µl de tampon Tris-HCl 50 mM, NaCl 150 mM, $CaCl_2$ 5 mM pH 7,5 et centrifugé à nouveau dans les mêmes conditions. Cette étape est répétée 3 fois permettant ainsi d'éliminer totalement le rouge de phénol.

20 µl de milieux conditionnés sans rouge de phénol sont ajoutés à 170 µl de tampon Tris-HCl 50 mM, NaCl 150 mM, $CaCl_2$ 5 mM pH 7,5 en plaque noire de 96 puits dont les sites de liaisons non-spécifiques ont été bloqués à l'aide d'une solution de sérum albumine bovine (SAB) à 0,1 % (p/v) dans un tampon Tris-HCl 50 mM, NaCl 150 mM,

$CaCl_2$ 5 mM pH 7,5. La réaction est initiée par addition de 10 µl de substrat Mca-Pro-Leu-Gly-Leu-(Dnp)-Ala-Arg-$NH_2$ à la concentration finale de 2 µM dans un volume réactionnel final de 200 µl. Les variations de fluorescence (longueur d'onde d'excitation : 326 nm, longueur d'onde d'émission : 465 nm) au cours du temps sont suivies à l'aide d'un spectrofluorimètre lecteur de plaque BMG Polarstar à +20°C. La courbe représentant la fluorescence (en RFU) en fonction du temps (en minutes) est tracée.

La vitesse initiale de la réaction est déterminée par le calcul de la pente de la tangente à l'origine. Le rapport $V_i/V_0$ est calculé.

**2.3. Modulation de l'activité MMPs en présence d'effecteur**

Activation de la proMMP-9

La proMMP-9 est activée par incubation pendant 18 heures à +4°C en présence de 1 mM d'Acide Phényl Mercurique Acétate (APMA), préparé à la concentration de 10 mM dans la soude 0,1 M).

Mesure de l'activité

200 pM de MMP-9 sont pré-incubés à +20°C pendant 5 minutes en absence ou en présence des différents candidats dans un tampon Tris-HCl 50 mM, NaCl 150 mM, $CaCl_2$ 5 mM pH 7,5 en plaque 96 puits noire dont les sites de liaisons non-spécifiques ont été bloqués à l'aide d'une solution de SAB à 0,1 % (p/v) dans le même tampon. La réaction est initiée par addition de 10 µl de substrat spécifique à la concentration finale de 2 µM dans un volume réactionnel final de 200 µl. Les variations de fluorescence (longueur d'onde d'excitation : 326 nm, longueur d'onde d' émission : 465 nm) au cours du temps sont suivies à l'aide d'un spectrofluorimètre lecteur de BMG Polarstar à +20°C. La courbe représentant la fluorescence (en RFU) en fonction du temps (en minutes) est tracée.
La vitesse initiale de la réaction est déterminée par le calcul de la pente de la tangente à l'origine. Le rapport $V_i/V_0$ est calculé.

**2.4. Résultats**

Les résultats sont représentés sur les figures 4 à 6. Sur les figures 5 et 6, on peut observer un effet dose, la dose étant indiquée en nanomolaires en abscisse après le nom de l'aptamère.

**Exemple 3 : Compositions selon la présente invention**

**3.1. Exemple A : Poudre cosmétique pour l'éclat du teint du visage**

| | |
|---|---|
| Microcellulose | 20,00% |
| Sodium lauryl sulfoacetate | 15,00% |
| Aptamère selon l'invention | 0,01% |
| Parfum, colorants, conservateurs | qs. |
| Talc | Qsp. 100% |

Cette poudre présente une double action. Elle permet d'une part un nettoyage de la peau, et d'autre part d'éclaircir le teint par un usage régulier durant quelques jours. On peut l'appliquer sur la peau du visage une à deux fois par jour.

**3.2. Exemple B : Crème cosmétique de jour anti-âge sous forme d'émulsion-gel**

| | |
|---|---|
| Glycérine | 5,00% |
| triglycérides caprylique/caprique/succinique | 5,00% |

(suite)

| | |
|---|---|
| Méthoxycinnamate d'octyle | 7,50% |
| Butyl méthoxydibenzoyle méthane | 2,00% |
| Diméthicone copolyol | 0,50% |
| Hyaluronate de sodium | 0.1% |
| Acrylates / C10-30 alkyl acrylate crosspolymer | 0,50% |
| Aptamère selon l'invention | 0,01% |
| Extrait de mauve | 3% |
| Neutralisant | qs. |
| Conservateurs, parfum, colorants | qs. |
| Eau | qsp. 100% |

Certaines personnes soumises au rayonnement plus ou moins intense de la lumière du jour, voire du soleil directement, souhaitent s'en protéger et éviter l'élastose solaire. L'utilisation de l'émulsion-gel de l'Exemple B permet d'atteindre ce but. Cette composition s'applique sur le visage préférentiellement e matin. Elle agit aussi bien de manière préventive que curative du photo-vieillissement, régulière ou non, du visage.

**3.3. Exemple C : Composition cosmétique fluide protectrice du rayonnement solaire (SPF 30)**

| | |
|---|---|
| Pentacyclométhicone volatile | 49,00% |
| Dioxyde de titane | 15,00% |
| Méthoxycinnamate d'octyle | 7,50% |
| Glycérine | 5,00% |
| Phényltriméthicone | 5,00% |
| Diméthicone copolyol | 3,00% |
| Polyméthylméthacrylate | 2,50% |
| Butyl méthoxydibenzoyle méthane | 1,00% |
| Aptamère selon l'invention | 0,01% |
| Neutralisant, Parfum, Conservateurs, antioxydants | qs. |
| Eau | qsp. 100% |

Cette composition est à utiliser avant l'exposition à un rayonnement solaire intense. Elle prévient l'apparition des rides, chez les personnes prédisposées à ce phénomène.

**3.4. Exemple D : Crème dermatologique pour le traitement anti-âge de nuit**

| | |
|---|---|
| Glycéryl stéarate + Peg-100 stéarate | 5,00% |
| Polyisobutène hydrogéné | 4,00% |
| Magnésium ascorbyl phosphate | 3,00% |
| Tricaprylate / caprate de glycérol | 3,00% |
| Squalane | 3,00% |

(suite)

| | |
|---|---|
| Glycérine | 3,00% |
| Beurre de karité | 1,50% |
| Cétéaryl octanoate | 1,50% |
| Ergothioneine | 0,50% |
| Alcool cétylique | 1,00% |
| Alcool stéarylique | 1,00% |
| Diméthicone | 1,00% |
| Gomme xanthane | 0,30% |
| Acide citrique | 0,10% |
| Citrate de sodium | 0,10% |
| Aptamère selon l'invention | 0,001% |
| Adenosine | 1,00% |
| Neutralisant, Parfum, Conservateurs | qs. |
| Eau | qsp. 100% |

L'utilisation de cette crème permet d'atténuer les rides et ridules par la synthèse de collagène, son action anti-oxydante et la protection de la matrice extra-cellulaire. Cette crème permet également d'atténuer les contrastes de couleur cutanée apparaissant avec l'âge.

**3.5. Exemple E : Lotion cosmétique visage anti-âge**

| | |
|---|---|
| Alcool éthylique | 5,00% |
| PPG-3 Myristyl éther | 1,00% |
| Glycérine | 3,00% |
| Carbomer | 0,20% |
| Polysorbate 20 | 0,20% |
| Tocopheryl phosphate de sodium | 0,1% |
| Biosaccharide gum 4 | 0,1% |
| Aptamère selon l'invention | 0,0001% |
| Extrait soja | 0,50% |
| Polyacrylate de sodium | 0,50% |
| Neutralisant, Parfum, Conservateurs | qs. |
| Eau | qsp. 100% |

Cette lotion, qui permet de combattre le vieillissement et le relâchement cutané, s'utilise après le démaquillage et le nettoyage de la peau.

**3.6. Exemple F : Sérum cosmétique anti-âge pour le visage**

| | |
|---|---|
| Eau | qsp 100% |
| Glycerine | 5,00% |
| EDTA tetrasodique Acide citrique Citrate trisodique | qsp pH souhaité |
| Gomme xanthane | 0,25% |
| Polyacrylamide, C13.14 isoparaffin, laureth-7 | 0,50% |
| Dimethicone copolyol | 0,25% |
| Aptamère selon l'invention | 1,00% |
| Adenosine | 1,00% |
| Extrait de mauve | 3,00% |
| Hyaluronate de sodium | 0,10% |
| Tocopheryl acetate | 0,20% |
| Polysilicone 11 | 1,00% |
| Pentacyclomethicone | 4,00% |
| Parfum, colorant, conservateur | qs |

Une goutte de cette composition très concentrée de sérum, s'applique sur le visage préférentiellement avant l'application d'une crème pour le visage. Ce sérum s'utilise préférentiellement par cures d'une à deux semaines pour un rajeunissement et lissage du teint.

### 3.7. Exemple G : Gel crème cosmétique anti-âge pour les mains

| | |
|---|---|
| Caprilic/capric diglyceryl succinate | 6% |
| Octyl octanoate | 2,5% |
| Methoxycinnamate d'octyle | 6% |
| Aptamère selon l'invention | 0,1% |
| Phenyltriméticone | 2,5% |
| Benzophenon-3 | 0,5% |
| Nitrure de bore | 1,00% |
| Huile de Camelia | 1% |
| Hyaluronate de sodium | 0,05% |
| Gomme xanthane | 0,2% |
| Acrylates/C10.30 alkyl acrylate copolymer | 0,5% |
| Glycerine | 7% |
| PEG 150 | 3% |
| Neutralisants, Colorants, parfum, conservateurs | Qs |
| Eau purifiée | qsp 100% |

Cette crème pour les mains protectrice des UV permet de prévenir l'apparition des rides et de lisser la surface cutanée.

**REFERENCES**

Inomata S et al., J. Invest. Dermatol., 2003, 120, 128-134.

Bertin et al., Journal of Medicinal Chemistry, 2005, Vol. 48, No. 24.

Lateef et al., American Journal of Pathology, Vol. 165, No. 1, July 2004.

Williamson et al., Cell, 1989, 59(5): 871-80.

Sundquist et Klug, Nature, 1989, 342(6251): 825-9.

Tucker et al., Curr Pharm Des., 2012, 18(14):2014-26.

Folgueras et al, Int. J. Dev. Biol., 2004, 48: 411 - 424.

Chaussain-Miller et al, J Dent Res., 2006, 85, 22-32.

Knight et al., Biochem J., 1991 Feb 15;274 (Pt 1):45-8.

Knight et al., FEBS Lett., 1992 Jan 27;296(3):263-6.

Williams and Bartel, Nucleic Acids Res.1995; (20) 4220-21).

Gu et al., Journal of Neuroscience, vol. 25, n°27, 6 juillet 2005, pages 6401 - 6408.

Gatto et al., Current Medicinal Chemistry, Vol.16, n°10, 1 avril 2009, pages 1248-1265.

Da Rochas Gomes et al., Bioconjugate Chemistry, vol.23, n°11, 21 novembre 2012, pages 2192-2200.

## Revendications

1. Aptamère à structure G-quadruplexe, capable d'inhiber l'activité enzymatique de la protéine MMP-9, **caractérisé en ce qu'**il s'agit d'un aptamère d'ADN comprenant au moins une séquence nucléotidique choisie dans le groupe consistant en la séquence consensus 5'$X_1X_2X_3X_4$TTTGGTGGGTYTGGGGTWGYK$X_5X_6$3', où X représente 0 ou 1 nucléotide G (SEQ ID N°1) ou l'une des séquences nucléotidiques SEQ ID NO :11 à SEQ ID NO 17, SEQ ID NO 32 à SEQ ID NO 34, et SEQ ID NO 40 à SEQ ID NO 45 comprises dans la SEQ ID N°1.

2. Aptamère selon la revendication 1, résistant aux nucléases.

3. Aptamère selon la revendication 1 ou 2, **caractérisé en ce que** ladite séquence nucléotidique comprend au moins 1 à 24 nucléotides contigus de la séquence SEQ ID NO :30 flanquée à son extrémité 5', et/ou au moins 1 à 23 nucléotides contigus de la séquence SEQ ID NO :31 flanquée à son extrémité 3'.

4. Aptamère selon l'une des revendications 1 à 3, **caractérisé en ce que** ladite séquence nucléotidique est choisie dans le groupe consistant en les séquences SEQ ID N0 : 12, SEQ ID N0 : 32, et SEQ ID N0 : 40.

5. Composition cosmétique ou pharmaceutique comprenant en tant que principe actif un aptamère selon l'une quelconque des revendications 1 à 4 en une quantité suffisante pour inhiber l'activité enzymatique de la protéine MMP-9 et un ou plusieurs excipients cosmétiquement ou pharmaceutiquement acceptables.

6. Composition selon la revendication 5, **caractérisée en ce qu'**elle comprend de 0,000001% à 10%, de préférence de 0,000002 à 5 %, de manière encore préférée de 0,000005 à 1% en poids de la composition d'un ou plusieurs aptamères selon l'une quelconque des revendications 1 à 5.

7. Utilisation cosmétique d'un aptamère selon l'une quelconque des revendications 1 à 4, pour traiter ou prévenir l'apparition des signes, visibles ou non, du vieillissement cutané intrinsèque et/ou extrinsèque ou pour en ralentir ou atténuer les effets, préférentiellement pour contrôler le remodelage cutané, restructurer l'épiderme, raffermir la peau, et/ou prévenir ou favoriser l'atténuation ou la résorption des rides.

8. Aptamère selon l'une quelconque des revendications 1 à 4, en tant que médicament.

9. Aptamère selon l'une quelconque des revendications 1 à 4 et 8, pour leur utilisation dans le traitement et/ou la prévention de pathologies choisies parmi le groupe consistant en des maladies de la peau préférentiellement sélectionnées parmi les maladies inflammatoires de la peau telles que le psoriasis, les tumeurs cutanées telles le carcinome basocellulaire, les lésions cutanées telles que les plaies chroniques, les pathologies de la cicatrisation, les dermatoses bulleuses telles que la pemphigoïde bulleuse, les maladies granulomateuses non infectieuses telles que la sarcoïdose de la peau, le granulome annulaire et la nécrobiose lipoïdique, et les dommages de la peau liés aux rayons ultra-violets.

10. Aptamère selon l'une des revendications 1 à 4 et 8, pour leur utilisation dans le traitement et/ou la prévention de pathologies choisies parmi le groupe consistant en en les pathologies tumorales, l'asthme, l'emphysème pulmonaire, la silicose, la bronchectasie, le purpura anaphylactoïde, le syndrome de détresse respiratoire aiguë (SDRA), l'arthrite rhumatoïde, la périodontite, les maladies intestinales inflammatoires, le lupus néphrétique, le syndrome de Sjögren, l'artérite à cellules géantes, l'anévrisme, les traumatismes des nerfs périphériques, la maladie d'Alzheimer, le syndrome de Guillain-Barré, la fibrose kystique, et les méningites.

11. Procédé de sélection d'un aptamère selon l'une quelconque des revendications 1 à 4 comprenant les étapes suivantes :

    - sélection dans une banque d'oligonucléotides d'aptamères par la méthode SELEX contre le site catalytique de la protéine MMP-9,
    - évaluation du potentiel à inhiber l'activité enzymatique de la protéine MMP-9 des aptamères identifiés à l'étape précédente,
    - clonage et séquençage des aptamères ainsi sélectionnés.

## Patentansprüche

1. Aptamer mit einer G-Quadruplex-Struktur, das fähig ist, die Enzymaktivität des MMP-9-Proteins zu hemmen, **dadurch gekennzeichnet, dass** es sich um ein DNA-Aptamer handelt, das zumindest eine Nukleotidsequenz umfasst, die aus der Gruppe bestehend aus der Konsensussequenz 5' $X_1X_2X_3X_4$TTTGGTGGGTYTGGGGTWGYK$X_5X_6$3' ausgewählt ist, wobei X 0 oder 1 G Nukleotid (SEQ ID NR: 1) oder eine der Nukleotidsequenzen SEQ ID NR: 11 bis SEQ ID NR: 17, SEQ ID NR: 32 bis SEQ ID NR: 34 darstellt, und SEQ ID NR: 40 bis SEQ ID NR: 45 in der SEQ ID NR: 1 enthalten sind.

2. Aptamer nach Anspruch 1, das resistent gegen Nukleasen ist.

3. Aptamer nach Anspruch 1 oder 2, **dadurch gekennzeichnet, dass** die Nukleotidsequenz zumindest 1 bis 24 angrenzende Nukleotide der an ihrem 5' Ende flankierten Sequenz SEQ ID NR: 30 und/oder zumindest 1 bis 23 angrenzende Nukleotide der an ihrem 3' Ende flankierten Sequenz SEQ ID NR: 31 umfasst.

4. Aptamer nach einem der Ansprüche 1 bis 3, **dadurch gekennzeichnet, dass** die Nukleotidsequenz aus der Gruppe bestehend aus den Sequenzen SEQ ID NR: 12, SEQ ID NR: 32 und SEQ ID NR: 40 ausgewählt ist.

5. Kosmetische oder pharmazeutische Zusammensetzung umfassend als Wirkstoff ein Aptamer nach einem der Ansprüche 1 bis 4 in ausreichender Menge, um die Enzymaktivität des MMP-9-Proteins zu hemmen, und einen oder mehrere kosmetisch oder pharmazeutisch verträgliche Hilfsstoffe.

6. Zusammensetzung nach Anspruch 5, **dadurch gekennzeichnet, dass** sie 0,000001 Gew.-% bis 10 Gew.-%, vorzugsweise 0,000002 bis 5 Gew.-%, weiter bevorzugt 0,000005 bis 1 Gew.-% der Zusammensetzung eines oder mehrerer Aptamere nach einem der Ansprüche 1 bis 5 enthält.

7. Kosmetische Verwendung eines Aptamers nach einem der Ansprüche 1 bis 4, um das Auftreten von sichtbaren oder nicht sichtbaren Anzeichen einer intrinsischen und/oder extrinsischen Hautalterung zu behandeln oder ihr vorzubeugen oder deren Auswirkungen zu verlangsamen oder abzuschwächen, vorzugsweise, um die Hautmodellierung zu kontrollieren, die Epidermis neu zu strukturieren, die Haut zu festigen und/oder um Falten vorzubeugen oder deren Milderung oder Rückbildung zu fördern.

8. Aptamer nach einem der Ansprüche 1 bis 4 als Arzneimittel.

9. Aptamer nach einem der Ansprüche 1 bis 4 und 8 zur Verwendung bei der Behandlung und/oder der Vorbeugung gegen Krankheiten, die aus der Gruppe bestehend aus Hautkrankheiten, die vorzugsweise aus entzündlichen Hautkrankheiten wie Psoriasis, Hauttumoren wie Basalzellkarzinom, Hautverletzungen wie chronische Wunden, Heilungsstörungen, bullöse Hautkrankheiten wie bullöses Pemphigoid, nicht ansteckende granulomatöse Krankheiten wie Hautsarkoidose, Granuloma anulare und Lipoidnekrobiose sowie Hautschäden im Zusammenhang mit ultravioletter Strahlung ausgewählt sind.

10. Aptamer nach einem der Ansprüche 1 bis 4 und 8 zur Verwendung bei der Behandlung und/oder der Vorbeugung gegen Krankheiten, die aus der Gruppe bestehend aus Tumorpathologien, Asthma, Lungenemphysem, Silikose, Bronchiektasie, anaphylaktoider Purpura, akutem Atemnotsyndrom (ARDS), rheumatoider Arthritis, Periodontitis, entzündlichen Darmerkrankungen, Lupusnephritis, Sjögren-Syndrom, Riesenzellarteriitis, Aneurysma, peripherem Nerventrauma, Alzheimer-Krankheit, Guillain-Barre-Syndrom, zystischer Fibrose und Meningitis ausgewählt sind.

11. Verfahren zum Auswählen eines Aptamers nach einem der Ansprüche 1 bis 4, das die folgenden Schritte umfasst:

    - Auswahl von Aptameren aus einer Oligonukleotidbank nach der SELEX-Methode gegen die katalytische Stelle des MMP-9-Proteins,
    - Potenzialbewertung der Hemmung der Enzymaktivität des MMP-9-Proteins der im vorherigen Schritt identifizierten Aptamere,
    - Klonen und Sequenzierung der somit ausgewählten Aptamere.

## Claims

1. G-quadruplex-structured aptamer, capable of inhibiting the enzymatic activity of MMP-9 protein, **characterized in that** it is a DNA aptamer comprising at least one nucleotide sequence selected from the group consisting of the consensus sequence 5'$X_1X_2X_3X_4$TTTGGTGGGTYTGGGGTWGYK$X_5X_6$3', where X represents 0 or 1 G nucleotide (SEQ ID NO: 1) or one of the nucleotide sequences SEQ ID NO: 11 to SEQ ID NO: 17, SEQ ID NO: 32 to SEQ ID NO: 34, and SEQ ID NO: 40 to SEQ ID NO: 45 included in SEQ ID NO: 1.

2. Aptamer according to claim 1, resistant to nucleases.

3. Aptamer according to claim 1 or 2, **characterized in that** said nucleotide sequence comprises at least 1 to 24 contiguous nucleotides of the sequence SEQ ID NO: 30 flanked at its 5' end, and/or at least 1 to 23 contiguous nucleotides of the sequence SEQ ID NO: 31 flanked at its 3' end.

4. Aptamer according to one of claims 1 to 3, **characterized in that** said nucleotide sequence is selected from the group consisting of the sequences SEQ ID NO: 12, SEQ ID NO: 32, and SEQ ID NO: 40.

5. Cosmetic or pharmaceutical composition comprising as active agent an aptamer according to any one of claims 1 to 4 in an amount sufficient to inhibit the enzymatic activity of MMP-9 protein and one or more cosmetically or pharmaceutically acceptable excipients.

6. Composition according to claim 5, **characterized in that** it comprises from 0.000001% to 10%, preferably from 0.000002% to 5%, more preferably from 0.000005% to 1% by weight of the composition of one or more aptamers according to any one of claims 1 to 5.

7. Cosmetic use of an aptamer according to any one of claims 1 to 4, for treating or preventing the appearance of signs, visible or not, of intrinsic and/or extrinsic skin aging or for slowing or attenuating the effects thereof, preferably for controlling skin remodeling, for restructuring the epidermis, for toning up the skin, and/or for preventing or promoting the smoothing or resorption of wrinkles.

8. Aptamer according to any one of claims 1 to 4, as a medicinal product.

9. Aptamer according to any one of claims 1 to 4 and 8, for use in treating and/or preventing pathologies selected from the group consisting of skin diseases preferably selected from inflammatory diseases of the skin such as psoriasis, skin tumors such as basal cell carcinoma, skin lesions such as chronic wounds, cicatrization pathologies, bullous dermatoses such as bullous pemphigoid, noninfectious granulomatous diseases such as sarcoidosis of the skin, granuloma annulare and necrobiosis lipedema, and skin damage related to ultraviolet rays.

10. Aptamer according to one of claims 1 to 4 and 8, for use in treating and/or preventing pathologies selected from the group consisting of tumor pathologies, asthma, pulmonary emphysema, silicosis, bronchiectasis, anaphylactoid purpura, acute respiratory distress syndrome (ARDS), rheumatoid arthritis, periodontitis, inflammatory intestinal disease, lupus nephritis, Sjögren syndrome, giant-cell arteritis, aneurysm, peripheral nerve trauma, Alzheimer's disease, Guillain-Barré syndrome, cystic fibrosis, and meningitis.

11. Method for selecting an aptamer according to any one of claims 1 to 4 comprising the following steps:

    - selecting in an oligonucleotide library, using the SELEX method, aptamers against the catalytic site of MMP-9 protein,
    - evaluating the potential to inhibit the enzymatic activity of MMP-9 protein of the aptamers identified in the preceding step,
    - cloning and sequencing the aptamers thus selected.

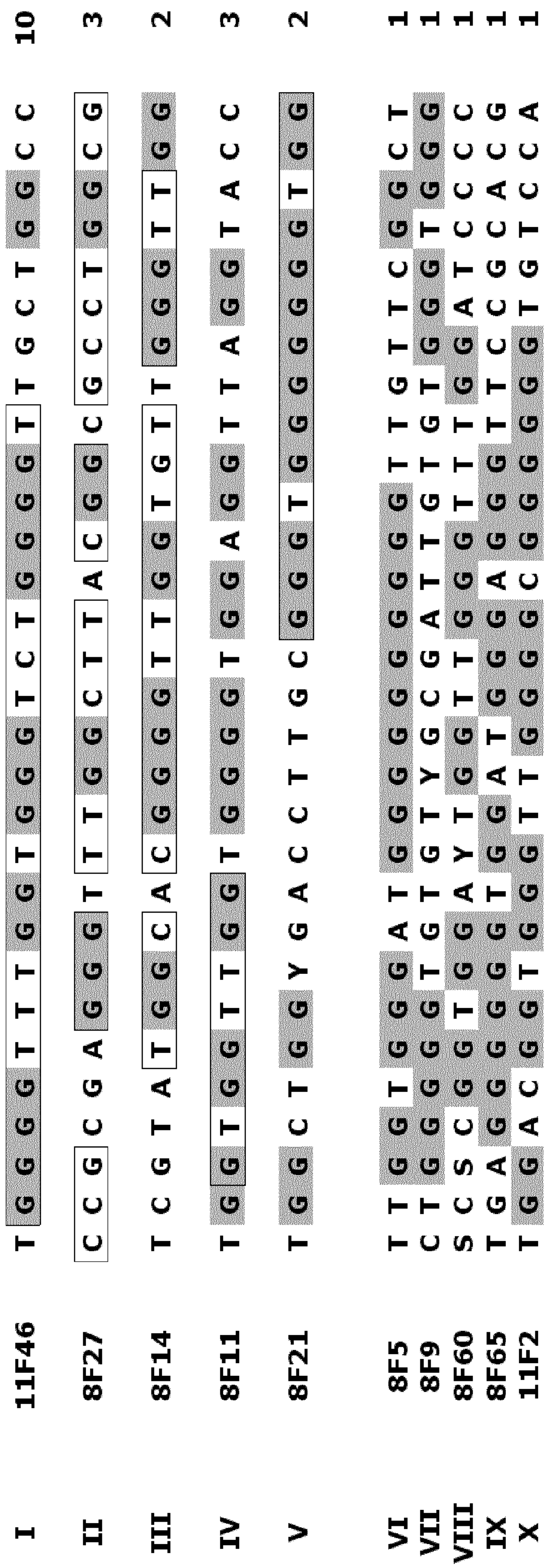
I 11F46 TGGGGTTTGGTGGGTCTGGGGTTGCTGGCC 10
II 8F27 CCGCGAGGGTTTGGCTTACGGCGCCTGGCG 3
III 8F14 TCGTATGGCACGGGGTTGGTGTTGGGTTGG 2
IV 8F11 TGGTGGTTGGTGGGGTGGAGGTTAGGTACC 3
V 8F21 TGGCTGGYGACCTTGCGGGTGGGGGGGTGG 2
VI 8F5 TTGGTGGGATGGGGGGGGGGTTGTTCGGCT 1
VII 8F9 CTGGGGGTGTGTYGCGATTGTGTGGGTGGG 1
VIII 8F60 SCSCGGTGGAYTGGTTGGGTTTGGATCCCC 1
IX 8F65 TGAGGGGGGTGGATGGGAGGGTTCCGCACG 1
X 11F2 TGGACGGTGGGTTGGGGCGGGGGGTGTCCA 1

Figure 1

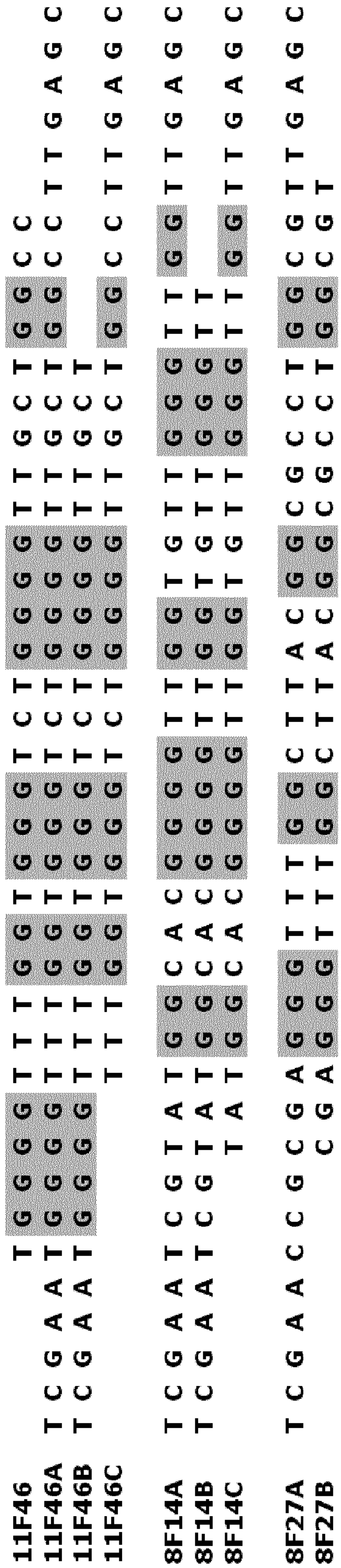
11F46 TGGGGTTTGGTGGGTCTGGGGTTGCTGGCC
11F46A TCGAATGGGGTTTGGTGGGTCTGGGGTTGCTGGCCTTGAGC
11F46B TCGAATGGGGTTTGGTGGGTCTGGGGTTGCT
11F46C TTTGGTGGGTCTGGGGTTGCTGGCCTTGAGC
8F14A TCGAATCGTATGGCACGGGGTTGGTGTTGGGTTGGTTGAGC
8F14B TCGAATCGTATGGCACGGGGTTGGTGTTGGGTT
8F14C TATGGCACGGGGTTGGTGTTGGGTTGGTTGAGC
8F27A TCGAACCGCGAGGGTTTGGCTTACGGCGCCTGGCGTTGAGC
8F27B CGAGGGTTTGGCTTACGGCGCCTGGCGT

Figure 2

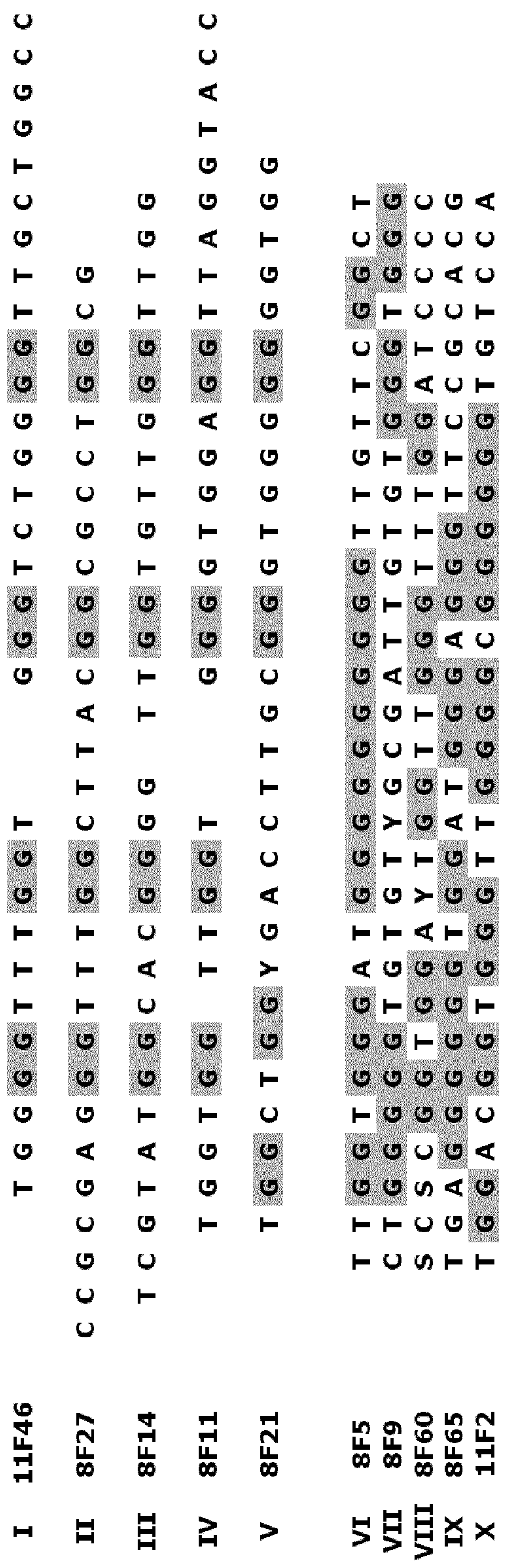

Figure 3

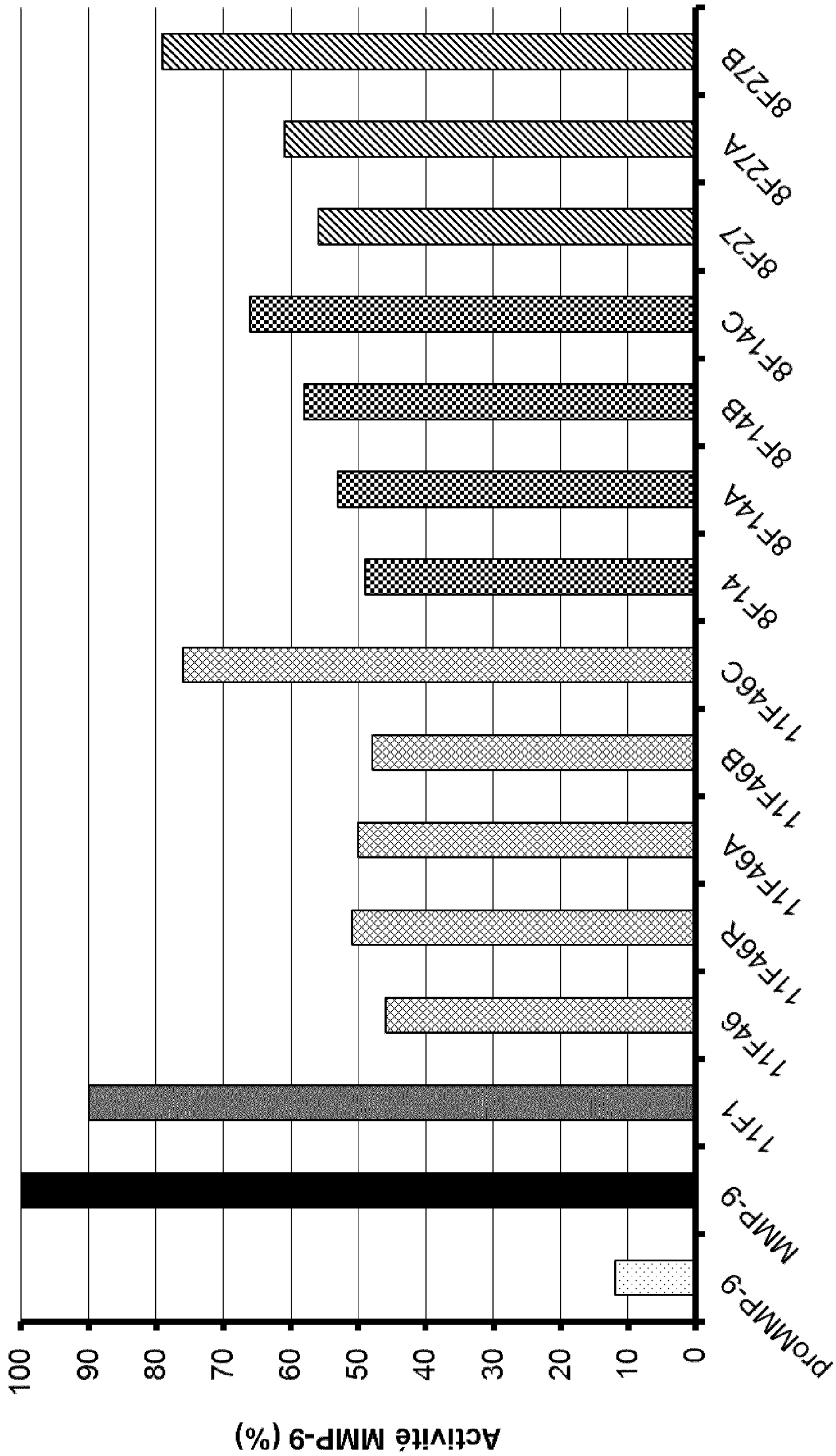
Activité MMP-9 (%)
0
10
20
30
40
50
60
70
80
90
100
proMMP-9
MMP-9
11F1
11F46
11F46R
11F46A
11F46B
11F46C
8F14
8F14A
8F14B
8F14C
8F27
8F27A
8F27B

Figure 4

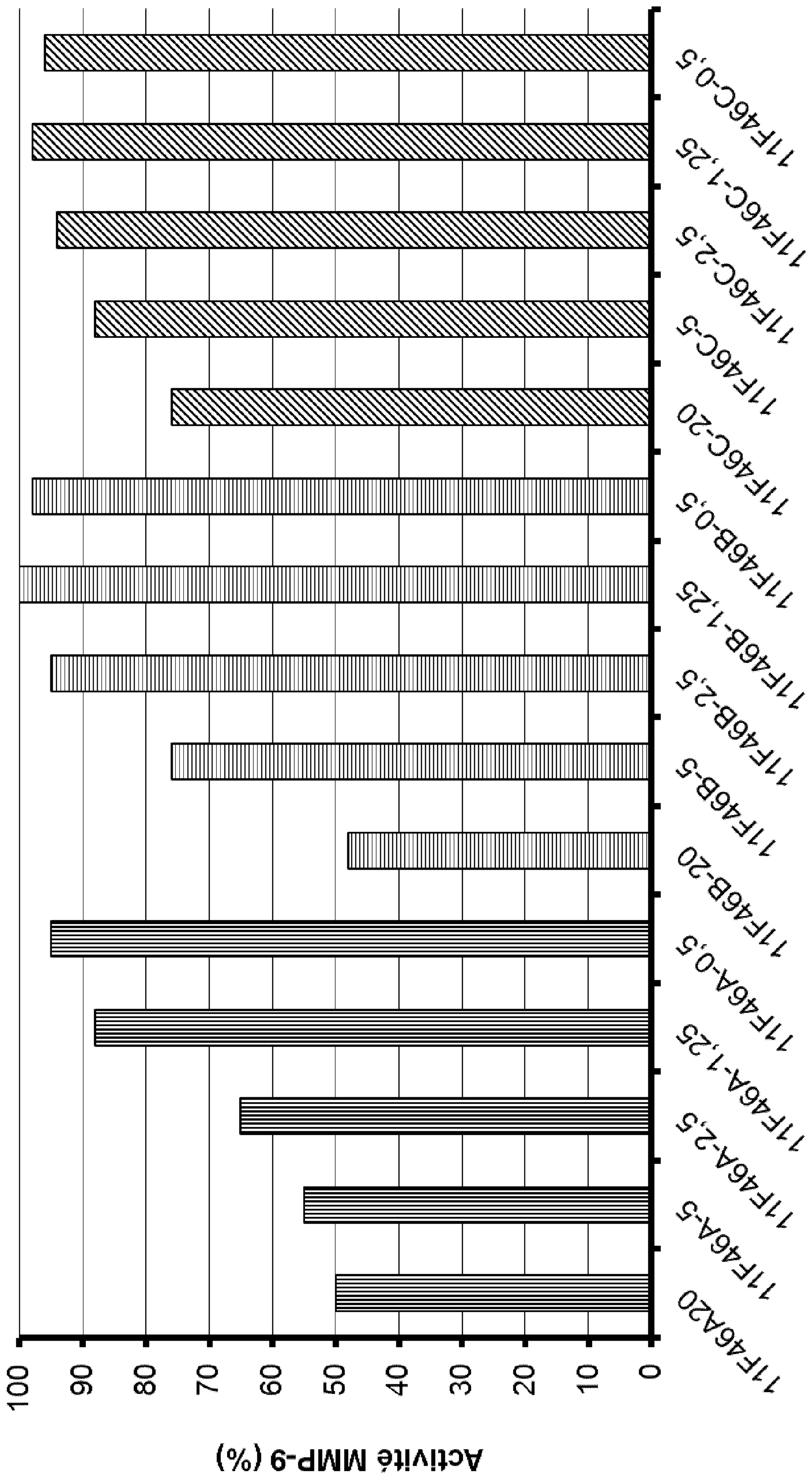
Activité MMP-9 (%)
0
10
20
30
40
50
60
70
80
90
100
11F46A20
11F46A-5
11F46A-2,5
11F46A-1,25
11F46A-0,5
11F46B-20
11F46B-5
11F46B-2,5
11F46B-1,25
11F46B-0,5
11F46C-20
11F46C-5
11F46C-2,5
11F46C-1,25
11F46C-0,5

**Figure 5**

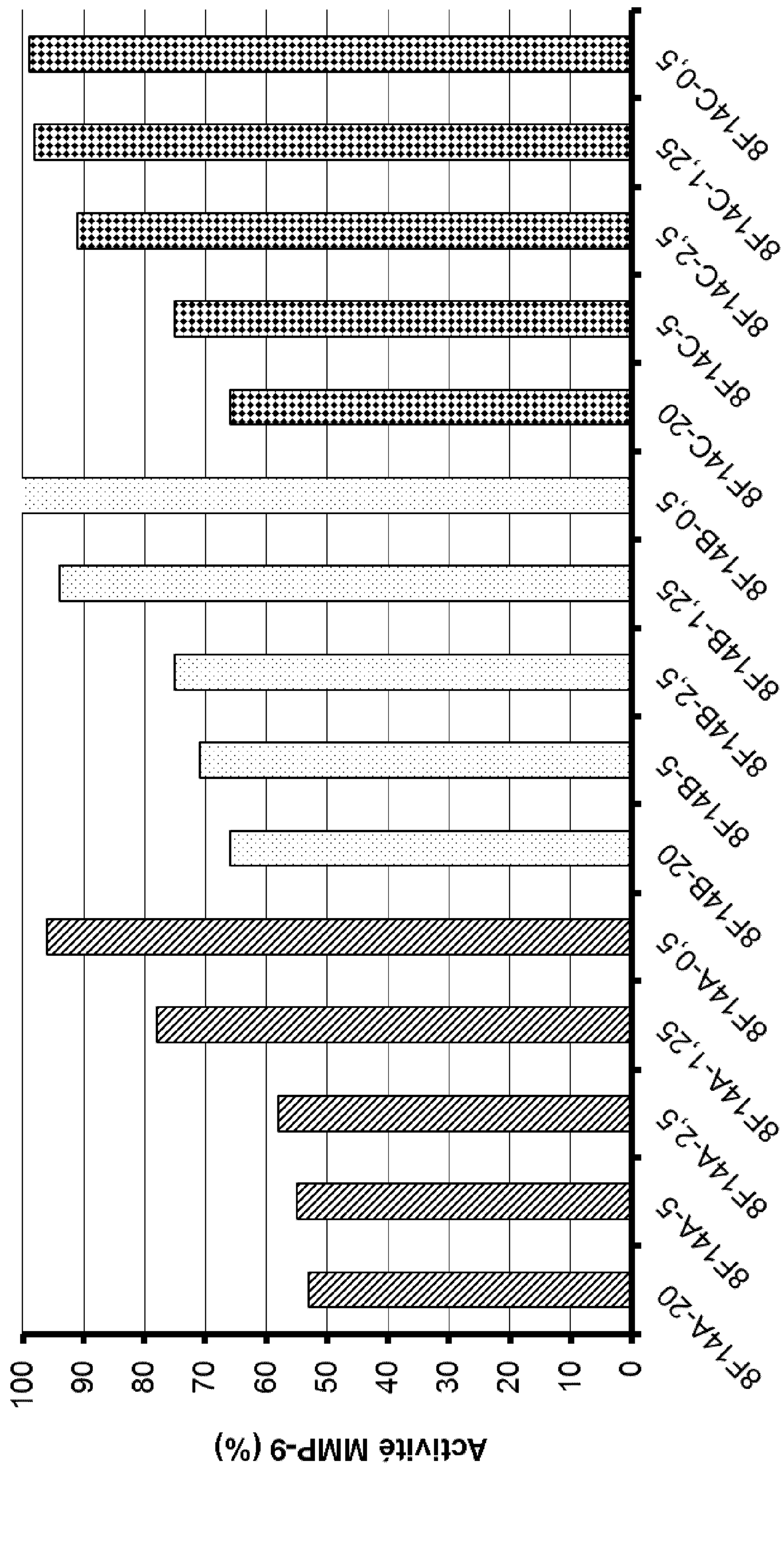
Activité MMP-9 (%)
0
10
20
30
40
50
60
70
80
90
100
8F14A-20
8F14A-5
8F14A-2,5
8F14A-1,25
8F14A-0,5
8F14B-20
8F14B-5
8F14B-2,5
8F14B-1,25
8F14B-0,5
8F14C-20
8F14C-5
8F14C-2,5
8F14C-1,25
8F14C-0,5

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2010110914 A2
- WO 2013153138 A
- WO 9119813 A
- FR 1363696


### Littérature non-brevet citée dans la description


- **INOMATA S et al.** *J. Invest. Dermatol.,* 2003, vol. 120, 128-134
- **BERTIN et al.** *Journal of Medicinal Chemistry,* 2005, vol. 48 (24
- **LATEEF et al.** *American Journal of Pathology,* Juillet 2004, vol. 165 (1
- **WILLIAMSON et al.** *Cell,* 1989, vol. 59 (5), 871-80
- **SUNDQUIST ET KLUG.** *Nature,* 1989, vol. 342 (6251), 825-9
- **TUCKER et al.** *Curr Pharm Des.,* 2012, vol. 18 (14), 2014-26
- **FOLGUERAS et al.** *Int. J. Dev. Biol.,* 2004, vol. 48, 411-424
- **CHAUSSAIN-MILLER et al.** *J Dent Res.,* 2006, vol. 85, 22-32
- **KNIGHT et al.** *Biochem J.,* 15 Février 1991, vol. 274 (1), 45-8
- **KNIGHT et al.** *FEBS Lett.,* 27 Janvier 1992, vol. 296 (3), 263-6
- **WILLIAMS AND BARTEL.** *Nucleic Acids Res.,* 1995, vol. 20, 4220-21
- **GU et al.** *Journal of Neuroscience,* 06 Juillet 2005, vol. 25 (27), 6401-6408
- **GATTO et al.** *Current Medicinal Chemistry,* 01 Avril 2009, vol. 16 (10), 1248-1265
- **DA ROCHAS GOMES et al.** *Bioconjugate Chemistry,* 21 Novembre 2012, vol. 23 (11), 2192-2200